# EUROPEAN PATENT SPECIFICATION

(11) **EP 3 011 055 B1**
(45) Date of publication and mention of the grant of the patent: **28.08.2019**
(21) Application number: 14813740.9
(22) Date of filing: 19.06.2014
(51) Int. Cl.: C12Q 1/6886, G01N 33/574

(54) **CLASSIFICATION SYSTEM, METHODS AND KIT FOR CLASSIFYING BREAST CANCER**
SYSTEM, VERFAHREN UND KIT ZUR KLASSIFIZIERUNG VON BRUSTKREBS
SYSTÈME DE CLASSIFICATION, PROCÉDÉ ET TROUSSE POUR LA CLASSIFICATION, D'UN CANCER DU SEIN

(30) Priority: 19.06.2013 US 201361836863 P
(43) Date of publication of application: 27.04.2016
(73) Proprietor: University Of Miami, Miami, FL 33136 (US)
(72) Inventor: INCE, Tan, A., Miami, FL 33132 (US)
(74) Representative: Schmidbauer, Andreas Konrad
(86) International application number: PCT/US2014/043128
(87) International publication number: WO 2014/205184

(56) References cited:
- WO-A1-2011/127219
- WO-A2-2012/092336
- US-A1- 2005 100 888
- US-A1- 2006 234 287
- US-A1- 2012 157 542
- US-A1- 2012 157 542
- SANDRO SANTAGATA ET AL: "Taxonomy of breast cancer based on normal cell phenotype predicts outcome", JOURNAL OF CLINICAL INVESTIGATION, vol. 124, no. 2, 27 January 2014 (2014-01-27), pages 859-870, XP055334416, US ISSN: 0021-9738, DOI: 10.1172/JCI70941
- ROBERT R. BURAS ET AL: "Vitamin D receptors in breast cancer cells", BREAST CANCER RESEARCH AND TREATMENT., vol. 31, no. 2-3, 1 January 1994 (1994-01-01), pages 191-202, XP055334455, US ISSN: 0167-6806, DOI: 10.1007/BF00666153
- KRISHAN, AWTAR ET AL.: 'Androgen and vitamin D receptor expression in archival human breast tumors.' CYTOMETRY PART B: CLINICAL CYTOMETRY vol. 58.1, 2004, pages 53 - 60, XP055302781
- PARISE CAROL A ET AL: "Breast Cancer Survival Defined by the ER/PR/HER2 Subtypes and a Surrogate Classification according to Tumor Grade and Immunohistochemical Biomarkers.", JOURNAL OF CANCER EPIDEMIOLOGY 2014, vol. 2014, 2014, page 469251, ISSN: 1687-8558
- KIM YOONSEOK ET AL: "Influence of Androgen Receptor Expression on the Survival Outcomes in Breast Cancer: A Meta-Analysis.", JOURNAL OF BREAST CANCER JUN 2015, vol. 18, no. 2, June 2015 (2015-06), pages 134-142, ISSN: 1738-6756
- SANDRO SANTAGATA ET AL: "Taxonomy of breast cancer based on normal cell phenotype predicts outcome", JOURNAL OF CLINICAL INVESTIGATION, vol. 124, no. 2, 27 January 2014 (2014-01-27), pages 859-870, US ISSN: 0021-9738, DOI: 10.1172/JCI70941
- ELEBRO KARIN ET AL: "Androgen receptor expression and breast cancer mortality in a population-based prospective cohort", BREAST CANCER RESEARCH AND TREATMENT, SPRINGER , NY, US, vol. 165, no. 3, 22 June 2017 (2017-06-22) , pages 645-657, ISSN: 0167-6806, DOI: 10.1007/S10549-017-4343-0 [retrieved on 2017-06-22]

## Description

### Statement regarding federally-sponsored research and development

This invention was made with U.S. government support under grant number R01-CA146445-01 awarded by the National Cancer Institute and the Breast Cancer Research Foundation. The U.S. government may have certain rights in the invention.

### BACKGROUND OF THE INVENTION

### Field of the Invention

This invention relates to disease classification, and more specifically to cancer and breast cancer classification. It further relates to methods of classifying diseases such as breast cancer and methods of treatment based on such classification.

### DESCRIPTION OF THE RELATED ART

Breast cancer is currently the second leading cause of death for women in the United States, and is the most commonly diagnosed cancer in women. Overall, 1 in 8 women in the U.S. will develop breast cancer in their lifetimes. Each year, over 40,000 women will die of breast cancer in the U.S. alone. Experts agree that early detection is one important element to better outcomes from breast cancer. However, in order to properly detect a disease, such as breast cancer, the medical community must first understand the disease and be able to recognize it in its various forms. It is therefore important to have an accurate and uniformly accepted classification system for particular diseases, in order to permit accurate diagnosing and treatment development.

While there are many ways to classify diseases, over the past two centuries morphology and function of normal tissue has been successfully used as a reference point to define various diseases of the same tissues. Most notably, such an approach has been used to effectively classify hematopoietic tumors such as lymphomas and leukemias (1). The discovery of the morphologic and molecular resemblance of various subtypes of leukemias and lymphomas to particular normal hematopoietic cell types was critical in this process.

Some of the most notable and earliest strides against cancers have been made in the treatment of hematopoietic malignancies (2). While many factors have contributed to this relative success, the accurate classification of hematopoietic malignancies played an important role. The relatively easy access to hematopoietic cells and the identification of cell-type specific cluster of differentiation (**CD**) markers on the surface of these cells, permitted efficient immunophenotyping, or the process of classifying cells based on particular protein expression patterns as identified via antibody staining. (3). These CD markers were later used to identify lymphomas and leukemias with a phenotype that was nearly identical to a specific normal cell type, allowing the development of the current classification system of these diseases (4,5).

Despite the major successes in rationally classifying and treating hematological malignancies, the use of normal cell types to classify other types of solid tumors has not been widely emulated. A major reason for this has been a lack of understanding of the diversity of cell types in most solid tissues. Characterization of the normal cell subtypes in solid tissues has been challenging.

Specifically with breast cancer, until recently only two cell types have been morphologically described in the human breast - the inner luminal cells and the outer myoepithelial cells (6). This limited understanding of the cell types comprising the breast ducts has precluded the development of a normal cell type-based classification system. While there has been more recent interest in normal breast cell subtypes, this research has been difficult to correlate with existing human breast tumor phenotypes (7).

Currently, human breast cancers are clinically grouped into three categories based on the presence of estrogen receptor (ER+), and human epidermal growth factor receptor 2 (HER2+), or by their absence in triple-negative breast cancers (ER/PR/HER2-, TNBC). However, such current classification does not account for the great heterogeneity of various breast cancer subtypes, and therefore is an insufficient classification system.

However, more accurate classification of cancers, and breast cancers in particular, is needed. Medical and therapeutic science shows that breast cancer tumors can be dormant or asymptomatic for many years, up to as many as 15 or 20 years, and may resurface after this prolonged period of time. Stress and hormonal changes, such as menopause, have been known to bring on shifts in cancer malignancy and aggression. It would therefore be beneficial to have a way to more properly diagnose not only breast cancer, but also what type of breast cancer, so as to have better patient outcomes even despite asymptomatic conditions or remission, and to better inform which treatments would be most effective.

WO 2011/127219 A1 discloses that biomarkers can be assessed for diagnostic, therapy-related or prognostic methods to identify phenotypes, such as a condition or disease, or the stage or progression of a disease. Circulating biomarkers from a bodily fluid can be used in profiling of physiological states or determining phenotypes. These include nucleic acids, protein, and circulating structures such as vesicles. Biomarkers can be used for theranostic purposes to select candidate treatment regimens for diseases, conditions, disease stages, and stages of a condition, and can also be used to determine treatment efficacy. The biomarkers can be circulating biomarkers, including vesicles and microRNA.

US 2012/157542 A1 discloses a method of classifying a sample of a patient who suffers from or being at risk of developing cancer, said method comprising the steps of determining in said sample from said patient, on a non protein basis, the expression level of at least one gene encoding for a hormone receptor selected from the group comprising estrogen receptor, progesterone receptor and/or androgen receptor in said sample; comparing the one or more expression level(s) determined with one or more expression level(s) of one or more reference genes, and classifying the sample of said patient from the outcome of the comparison into one of at least two classifications.

ROBERT R. BURAS ET AL, "Vitamin D receptors in breast cancer cells", BREAST CANCER RESEARCH AND TREATMENT., US, 1994, vol. 31, no. 2-3, doi:10.1007/BF00666153, ISSN 0167-6806, pages 191-202, discloses that 1,25-(OH)₂-Vitamin D₃, the active metabolite of vitamin D, is a secosteroid hormone with known differentiating activity in leukemic cells. Studies have demonstrated the presence of vitamin D receptors (VDR) in a wide range of tissues and cell types. Antiproliferative activity of 1,25-(OH)₂-vitamin D₃ has been documented in osteosarcoma, melanoma, colon carcinoma, and breast carcinoma cells. This study was designed to analyze vitamin D receptor level in breast cancer cells as a marker, of differentiation and as a predictor of growth inhibition by 1,25-(OH)₂-vitamin D₃. VDR messenger RNA was found to be present in relatively high levels in well-differentiated cells and in low levels in poorly differentiated cells. All cell lines had detectable VDR mRNA. Radiolabeled ligand binding assay showed a similar pattern. MCF-7 and T47D cells, which express VDR at moderate levels, showed significant growth inhibition by 10⁻⁹ M 1,25-(OH)₂-vitamin D₃ (p < 0.05). MDA-MB-231 cells, which have very low levels of VDR, demonstrated no growth inhibition by 1,25-(OH)₂-vitamin D₃ at concentrations up to 10⁻⁶ M. Based on these results it can be stated that VDR expression is lost with dedifferentiation and that receptor is essential for the antiproliferative response to 1,25-(OH)₂-vitamin D₃.

WO 2012/092336 A2 discloses methods and systems of molecular profiling of diseases, such as cancer. In some embodiments, the molecular profiling can be used to identify treatments for a disease, such as treatments that were not initially identified as a treatment for the disease or not expected to be a treatment for a particular disease. The cancer can be an ovarian cancer.

### SUMMARY OF THE INVENTION

In order to better understand and treat breast cancer, a classification system for human breast cancer is provided based on normal breast cell phenotypes, according to the differentiation state of cell populations. An appropriate and accurate classification system is important in not only understanding breast cancer, but consequently in identifying and treating it. It therefore provides a strong foundation for breast cancer medicine.

The classification system is based on the comparison of breast cancer cells to normal breast cell phenotypes for classification, which has not been seen heretofore. An understanding of the normal breast cell subtypes is therefore important for the current invention, but characterizing normal breast cell subtypes in solid tissues has been challenging to date. The normal human breast is composed of milk producing lobules and interlobular ducts that transport the milk to the nipple. The anatomical distinction between ducts and lobules is important in order to understand the pathophysiology of breast cancer. The vast majority of human breast tumors are currently classified on morphological grounds either as -"ductal carcinomas" or as "lobular carcinomas" by pathologists for reasons that are unrelated to their cell-of-origin. Nevertheless, this arcane terminology has resulted in a common misconception that ductal and lobular breast cancers initiate in the normal ducts and lobules, respectively. However, despite their names, almost all of the early progression steps for both tumor types almost exclusively involve the breast lobules. Thus, specifically examining the normal cells in the lobules using methods such as immnunohistochemical (IHC) staining that preserves tissue architecture and allows discrimination of ducts, lobules and different layers of the epithelium was a first step in determining the classification system of the present invention.

Until recently only two cell types have been morphologically described in the human breast; the inner luminal cells and the outer myoepithelial cells (6). This limited understanding of the cell types comprising the breast ducts has precluded the development of a normal cell type-based classification system. To meet this need, the present invention relates to a way of characterizing normal breast cell subtypes, and breast cancer subtypes, in a way that has not been done before and which more accurately describes the breast cancer.

More in particular, the present invention provides a method of classifying breast cancer as set forth in claim 1, and the use of a kit in a method of classifying breast cancer as set forth in claim 8. Preferred embodiments of the present invention may be gathered from the dependent claims. The disclosed classification system for breast cancer based on normal breast cell phenotypes includes at least four categories (denoted herein as HR0-HR3) based at least on the expression profile of estrogen receptor (ER), androgen receptor (AR), and vitamin D receptor (VDR) within the tissue. A first category (HR0) includes tissues which do not express any of ER, AR, or VDR. The second category (HR1) encompasses tissues expressing only one of ER, AR, or VDR. The third category (HR2) includes tissues having any combination of two of ER, AR, and VDR. And a fourth category (HR3) includes tissues expressing all three of ER, AR, and VDR. Each of these four categories can be further broken down into subclasses based on the presence of certain other markers, such as pan-luminal markers K7, K8, K18, proliferating cell marker Ki67, and K5. Being able to better classify various types of breast cancers means better and more accurate diagnosis. Accordingly, the present invention includes a method of classifying breast cancer, which includes measuring the levels of ER, AR, and VDR expression in the tumor sample and classifying the type of breast cancer according to the classification system described herein.

Each of the four main categories of breast cancer based on ER/AR/VDR expression is associated with a different survival rate, allowing for the prediction of more accurate prognosis than is currently available. A method of predicting the prognosis of breast cancer is provided including measuring the levels of ER, AR, and VDR in the tumor or cancerous tissue, and determining a survival rate based on the classification system described herein. Specifically, patients having tumors that express all three ER, AR, and VDR have a better survival outcome than those that only express two of the hormone receptors, which still fare better than those that only express one of the hormone receptors. If no hormone receptors are expressed, the survival rate is less good during the first five years following diagnosis, but gets better thereafter.

Hormone treatment is provided for the corresponding receptors that are present or expressed in the cancerous tissue. In cases where more than one hormone receptor is present, a multi-hormone treatment regimen and administration is contemplated. This can be done independently of or in connection with chemotherapy and/or radiation treatments as well, depending on the severity and progression of the cancer.

These and other objects, features and advantages of the present invention will become clearer when the drawings as well as the detailed description are taken into consideration.

### BRIEF DESCRIPTION OF THE DRAWINGS

For a fuller understanding of the nature of the present invention, reference should be had to the following detailed description taken in connection with the accompanying drawings in which:

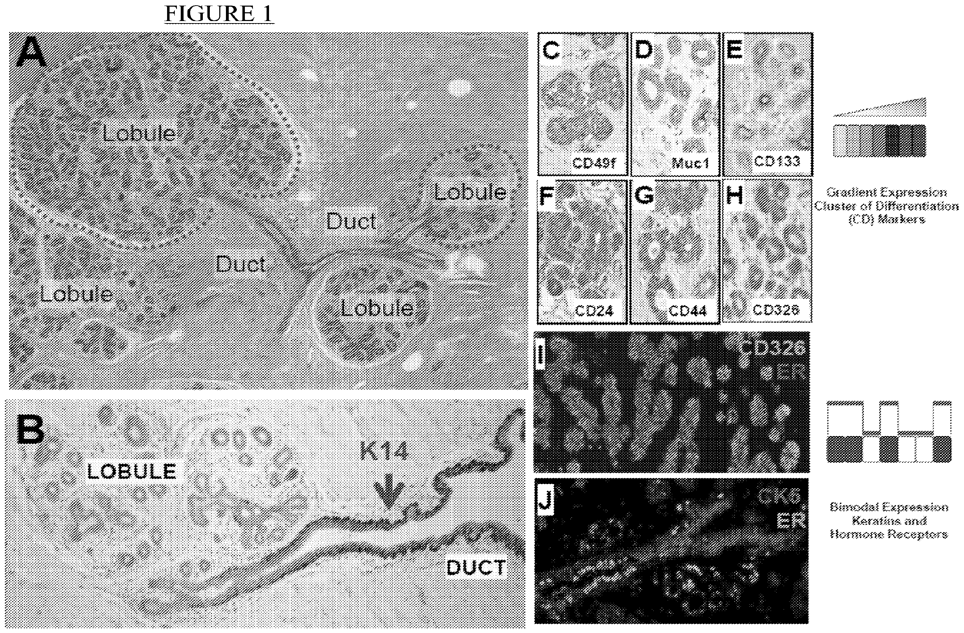

Figure 1 shows the anatomy of normal human breast and expression patterns of keratins, cluster differentiation (CD) markers and hormone receptors in normal human breast epithelium. **(A)** Hematoxylin and eosin (H&E) stained section of normal human breast epithelium, showing lobules and interlobular ducts. **(B)** Immunohistochemistry for keratin K14 on normal human breast epithelium. While the interlobular ducts stain with K14 (brown) in the myoepithelial cells only, the majority of myoepithelial cells in the lobules are negative for K14 in this section. **(C-H)** Immunohistochemistry for cluster differentiation markers makers CD49f, Muc1, CD133, CD24, CD44 and CD326 (Ep-CAM). These markers were positive in most luminal epithelial cells in the lobules and ducts. There was not differential expression between ducts and lobules. While some cells expressed lower levels and others higher levels, there were not distinct positive and negative populations. **(I)** Immunofluorescence staining for estrogen receptor (ER) (shown in red) and CD326 (shown in green) (Ep-CAM) shows that both ER[+] and ER[-] cells are CD326 [+]. Thus, there is no correlation between the expression of these two markers. **(J)** Immunofluorescence staining for ER (in green) and K5 (in red) shows that ER[+] and K5 [+] staining are bimodal (biphasic). Unlike the CD markers there was a clearly positive and a negative cell population for each marker, and ER[+] and K5 [+] were mutual exclusive, i.e., if a cell is K5[+] it is almost always ER[-], and vice versa.

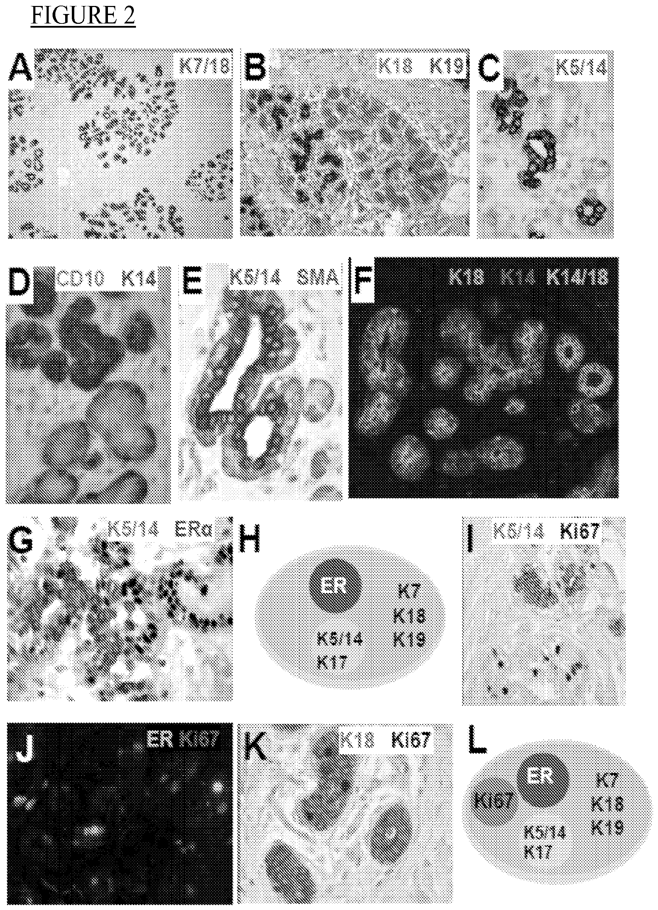

Figure 2 shows immunofluorescence images of the expression of intermediate filaments and estrogen receptor (ER) in normal human breast. Single and double immunostains of normal human formalin-fixed, paraffin-embedded (FFPE) sections with **(A)** K7/18 (brown), **(B)** K18 (red) and K19 (brown), **(C) K**5/14 (brown), **(D)** CD10 (red) and K14 (brown), **(E) K**5/14 (brown) and smooth-muscle actin (SMA-red), **(F) K**18 (green), K14 (red), cells expressing both K18 and K14 (yellow), **(G)** K5/14 (red) and estrogen receptor (ER-brown). This population of cells [K5+, K14+ or K17+] is designated because the tissue sections were not stained simultaneously with these markers. **(H)** Venn diagram showing differentiation states of normal luminal epithelial cells based on expression of ER and K5/7/14/17/18/19, (I) Ki-67 (brown) and K5/14 (blue), **(J)** estrogen receptor (ER-green) and proliferation marker Ki-67 (red), **(K) K**18 (red) and Ki-67 (brown), **(L)** Venn diagram showing differentiation states of normal luminal epithelial cells based on ER, Keratins and Ki-67. Immunoperoxidase (**A-E, G, I and K**), and merged images of immunofluorescence (**F, J**) stains are shown. Representative images are shown selected from multiple patient samples (n=36).

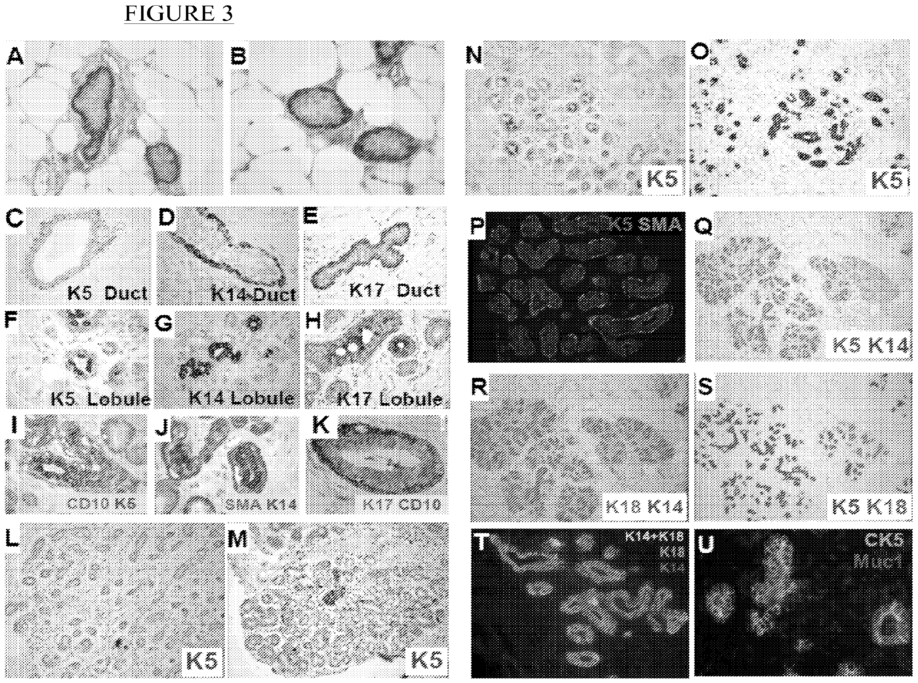

Figure 3 shows keratin 5, 14 and 17 expression in normal human mammary gland ducts versus lobules. **(A-B)** Keratin 5 immunostains of mouse mammary glands show that K5 is expressed only in the myoepithelial layers, unlike human breast that has abundant luminal K5 expression (K5=brown). **(C-E)** Keratins 5, 14 and 17 are expressed in the myoepithelial (basal) layer in the interlobular ducts of normal human breast tissue (K5, 14, 17 shown as brown, with blue counter stain). **(F-H)** Keratins 5, 14 and 17 are expressed in the luminal layer in the lobules of normal human breast tissue (K5, 14, 17 shown as brown, with blue counter stain). **(I-K)** Double immunostains with universal myoepithelial markers CD10 and SMA show that K5/14/17 cells are located above the CD10/SMA[+] myoepithelial cells next to the lumen. K5 shown in brown (I), K14 shown in brown (J), K17 shown in red (K), CD10 shown in red (I), SMA shown in red (J), CD10 shown in brown (K). **(L-P)** The frequency of K5+ cells can be dramatically different from lobule to lobule even in the same section from the same person. **(L)** In many lobules there are almost no K5+ cells. **(M)** In rare lobules K5 is expressed in some of the myoepithelial cells but not in luminal cells. **(N)** In many lobules nearly half of the luminal cells are K5+. **(O-P)** In some lobules almost all the luminal cells are K5+, either with single K5 stain (shown in brown, panel M), or with double immunostain that confirms the luminal nature of these cells (K5 shown in red, SMA shown in green, panel N). **(Q-S)** Double **i**mmunostaining of serial sections of the same lobule show that in some lobules nearly all cells are K5 and K18 double positive. K5 shown in red, K14 shown in brown (Q), K18 shown in red, K14 shown in brown (R), K5 shown in brown, K18 shown in blue (S). **(T)** Double immunostains show that in some lobules almost all luminal cells are double K18 and K14 positive. K18 shown in green, K14 shown in red, K14/18 double positive cells shown in yellow. **(U)** Most of the K5 luminal cells express Muc1, a marker of differentiation. K5 shown in green, Muc1 shown in red.

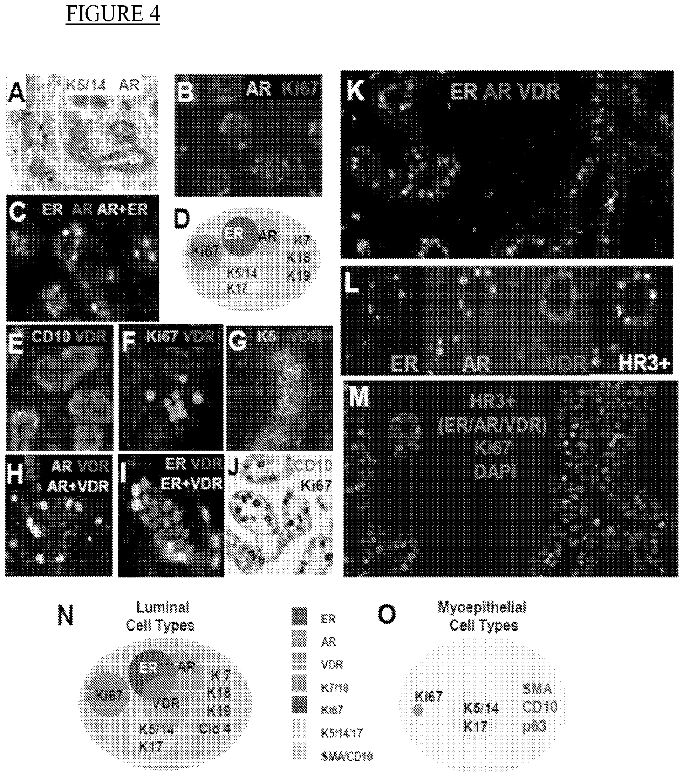

Figure 4 shows immunofluorescence images of the expression of intermediate filaments, estrogen receptor (ER), androgen receptor (AR) and vitamin D receptor (VDR) in normal human breast. Double immunostains (A, J) and merged immunofluorescence images (B, C, E-I, K-M) of normal human breast FFPE sections are shown. Venn diagrams show differentiation states of luminal (D, N) and myoepithelial (O) cell types. **(A)** K5/14 (red) and androgen receptor (AR, brown). **(B)** Androgen receptor (AR, green) and proliferation marker Ki-67 (red). **(C)** ER (green), AR (red) and double positive cells (yellow). **(D)** Venn diagram showing differentiation states of normal luminal epithelial cells based on ER, Keratins, Ki-67 and AR. **(E)** CD10 (green) and vitamin D receptor (VDR, red). **(F)** VDR (red) and Ki-67 (green). **(G)** K5 (green) and VDR (red). **(H)** AR (green), VDR (red) and double positive (yellow). **(I)** ER (green), VDR (red) and double positive (yellow). **(J)** CD10 (red) and Ki-67 (brown). **(K)** ER (green) AR (red) VDR (blue), ER+AR (yellow), ER+VDR (purple), VDR+ER (light blue), **(L)** Triple hormone receptor positive cells ER+AR+VDR (HR3+, white). **(M)** HR3+ (ER+AR+VDR) cells (green), proliferation marker Ki-67 (red), and nuclear marker DAPI (blue). **(N-O)** Venn diagram showing differentiation states of normal luminal **(N)** and myoepithelial **(O)** breast cells on based on the full marker panel. Representative images are shown selected from multiple patient samples (n=36).

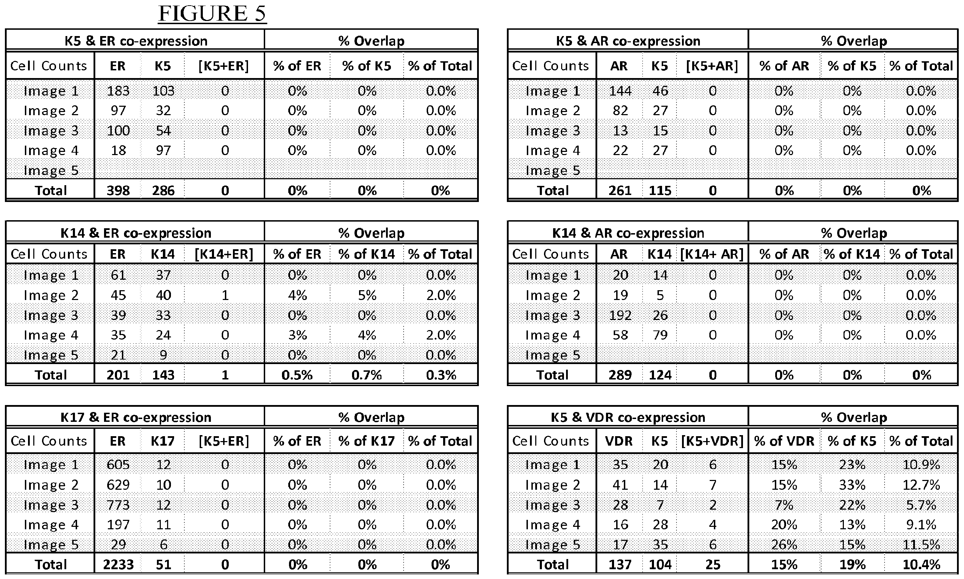

Figure 5 is a table showing the co-expression frequency of hormone receptors ER, AR and VDR with K14 and K5. Each row is a different normal breast sections stained with the indicated double immunostains. The total number of cells positive for each marker (column 1-2), both markers (column 3), the percentage of double-positive cells as a fraction of cells positive for each marker (column 4-5) and both markers (column 6) are depicted.

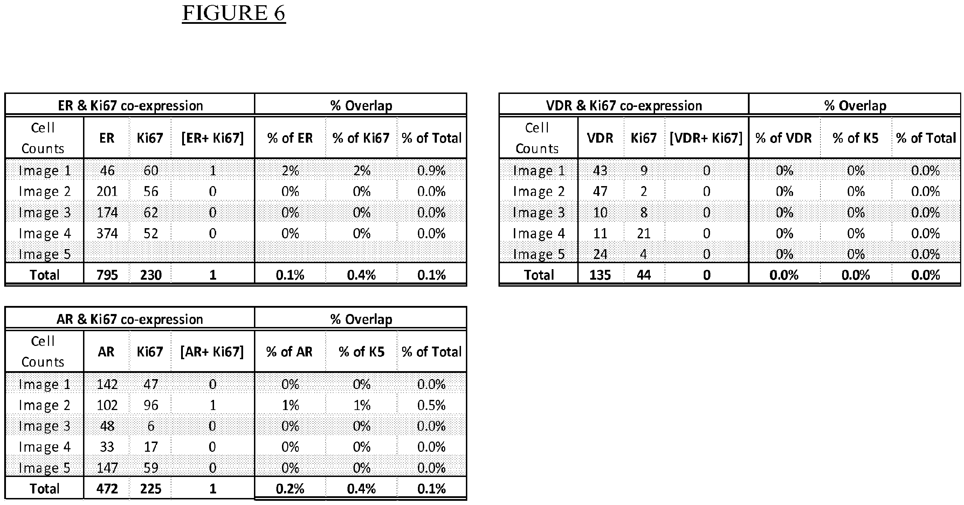

Figure 6 is a table showing the co-expression frequency of hormone receptors ER, AR, and VDR with proliferation marker Ki-67. Each row is a different normal breast sections stained with the indicated double immunostains. The total number of cells positive for each marker (column 1-2), both markers (column 3), the percentage of double-positive cells as a fraction of cells positive for each marker (column 4-5) and both markers (column 6) are depicted.

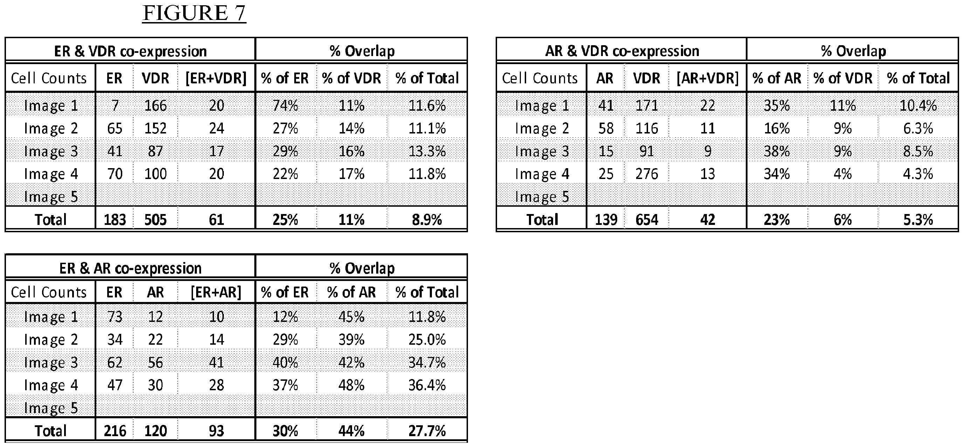

Figure 7 is a table showing the co-expression frequency of hormone receptors ER, AR and VDR in the luminal layer cells of normal human breast. Each row is a different normal breast sections stained with the indicated double immunostains. The total number of cells positive for each marker (column 1-2), both markers (column 3), the percentage of double-positive cells as a fraction of cells positive for each marker (column 4-5) and both markers (column 6) are depicted.

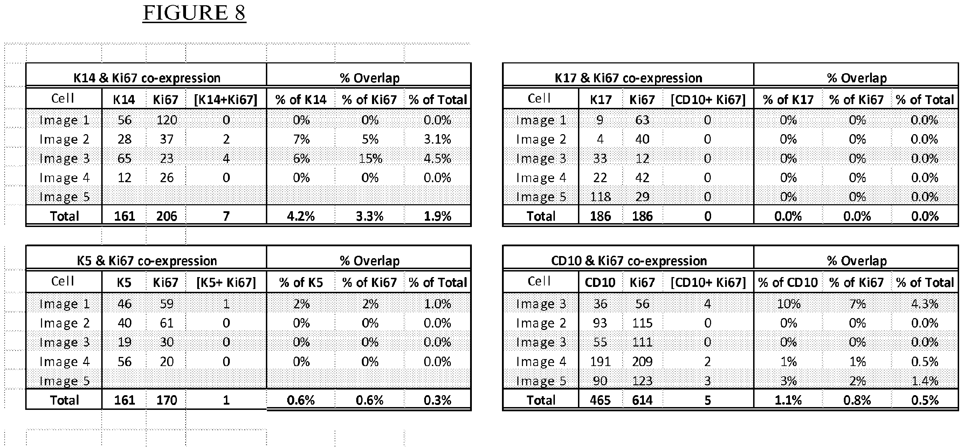

Figure 8 is a table showing the co-expression frequency of hormone receptors K14, K5, and CD10 with proliferation marker Ki-67. Each row is a different normal breast sections stained with the indicated double immunostains. The total number of cells positive for each marker (column 1-2), both markers (column 3), the percentage of double-positive cells as a fraction of cells positive for each marker (column 4-5) and both markers (column 6) are depicted.

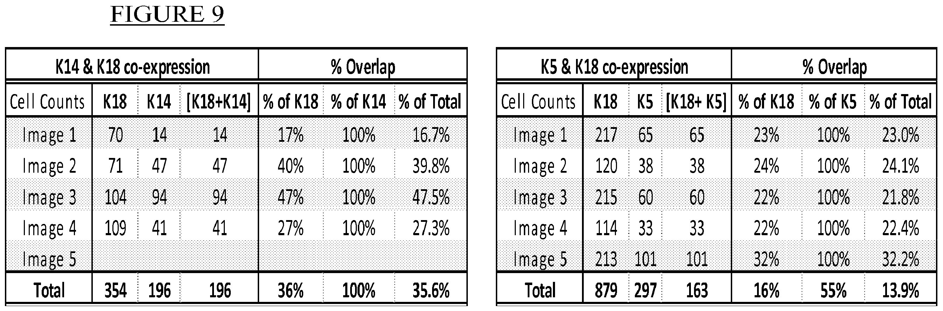

Figure 9 is a table showing the co-expression frequency of K14 and K5 with K18. Each row is a different normal breast sections stained with the indicated double immunostains. The total number of cells positive for each marker (column 1-2), both markers (column 3), the percentage of double-positive cells as a fraction of cells positive for each marker (column 4-5) and both markers (column 6) are depicted.

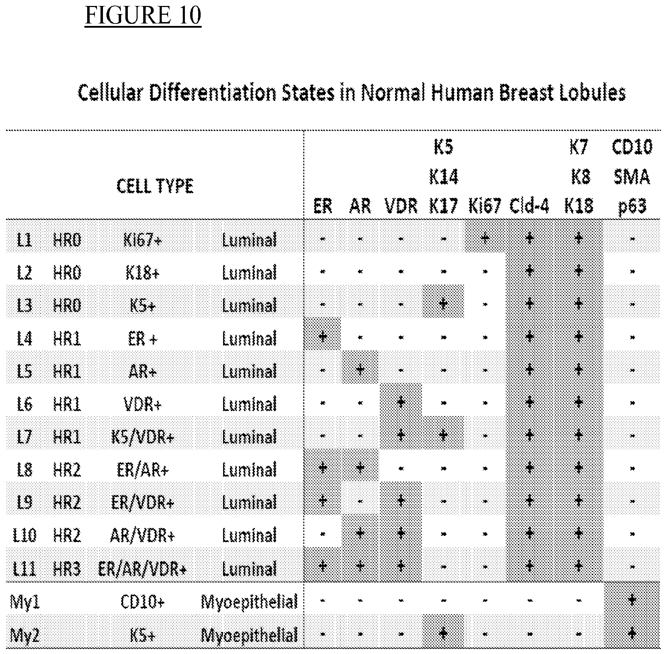

Figure 10 is a table showing the immunostaining results of normal human breast sections from multiple donors (n=36) with fourteen different markers identified multiple normal breast cell subtypes, and resulting grouping for classification based thereon. The 11 differentiation states in the luminal layer of human breast lobules (L1-11) are grouped into hormone receptor negative (HR0; L1-3), single hormone receptor positive (HR1+: ER, AR or VDR; L4-L6), double hormone receptor positive (HR2+: ER/AR, ER/VDR, or AR/VDR; L7-L10) and triple hormone receptor positive (HR3+: ER/AR/VDR; L11) states. All luminal cells expressed K7/18 and Cld-4. In the myoepithelial layer, all cells expressed SMA, CD10 and p63, with two subtypes that were either K5/14/17[-] or K5/14/17[+] (My1 and My2).

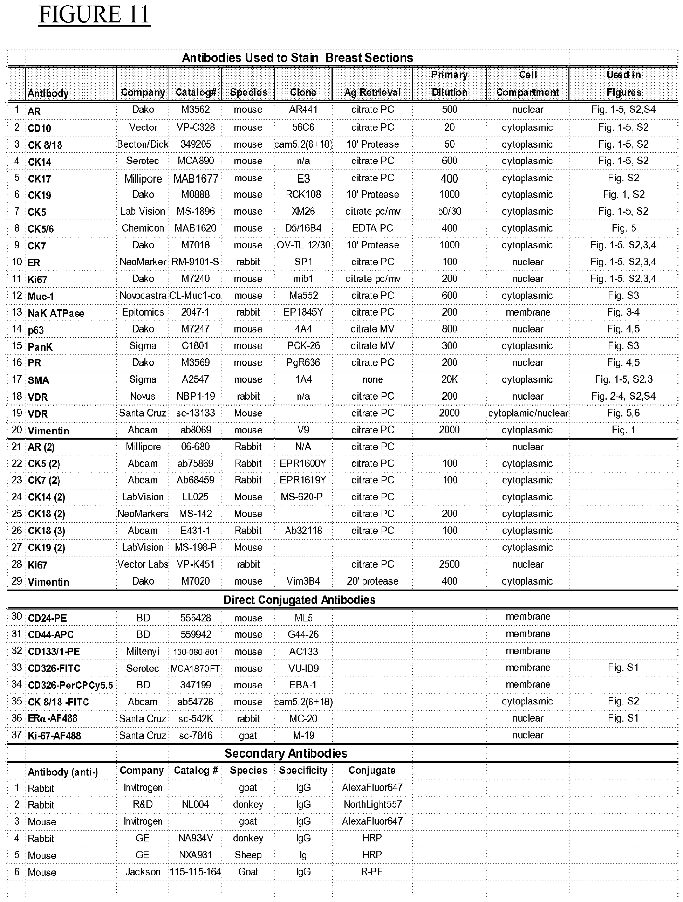

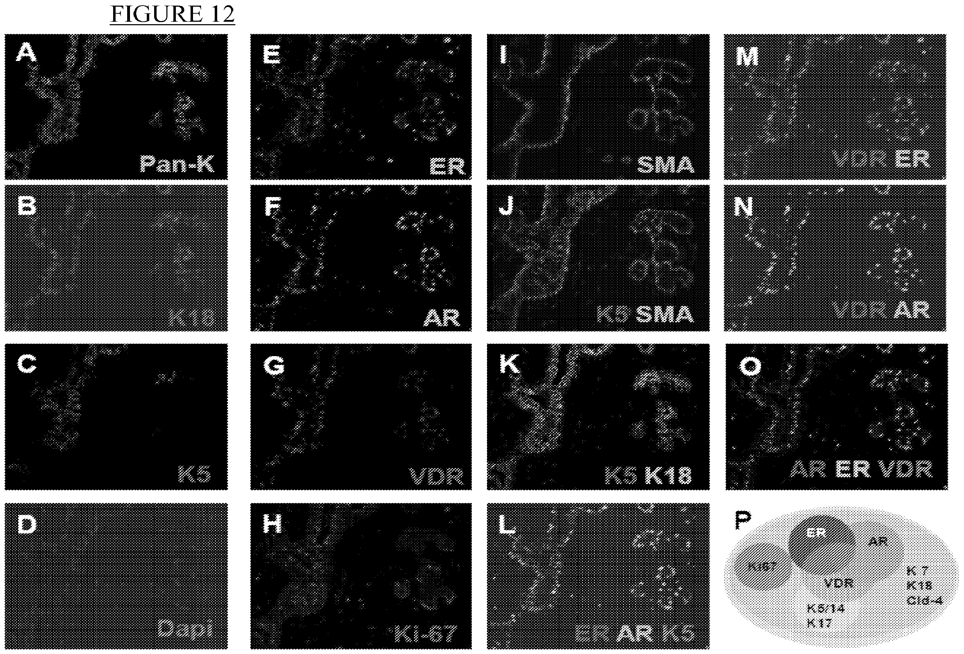

Figure 11 is a table listing the various antibodies used in generating the immunofluorescence data of Figure 12.
Figure 12 shows multiplex immunofluorescent examination of twelve different markers *in situ* in normal human breast. One FFPE section of normal breast epithelium was stained serially with each antibody for the indicated markers: **(A)** Pan-Keratin, **(B)** K18, **(C)** K5, **(D)** DAPI, **(E)** ER, **(F)** AR, **(G)** VDR, **(H)** Ki-67, **(I)** SMA. Individual immunofluorescence staining images **(A-I)** were merged **(J-O)** to reveal co-expression pattern of all markers in each cell: **(J)** K5 (red) and SMA (green), **(K)** K5 (red) and K18 (green), **(L)** ER (red), AR (green), K5 (blue), **(M)** VDR (red) and ER (green), **(N)** VDR (red) and AR (green), **(O)** AR (red) ER(green) VDR (blue). **(P)** Venn diagram showing differentiation states of normal luminal breast cells based on full marker panel. Representative images acquired with a multiplexed immunofluorescence technology (GE Healthcare) are shown.

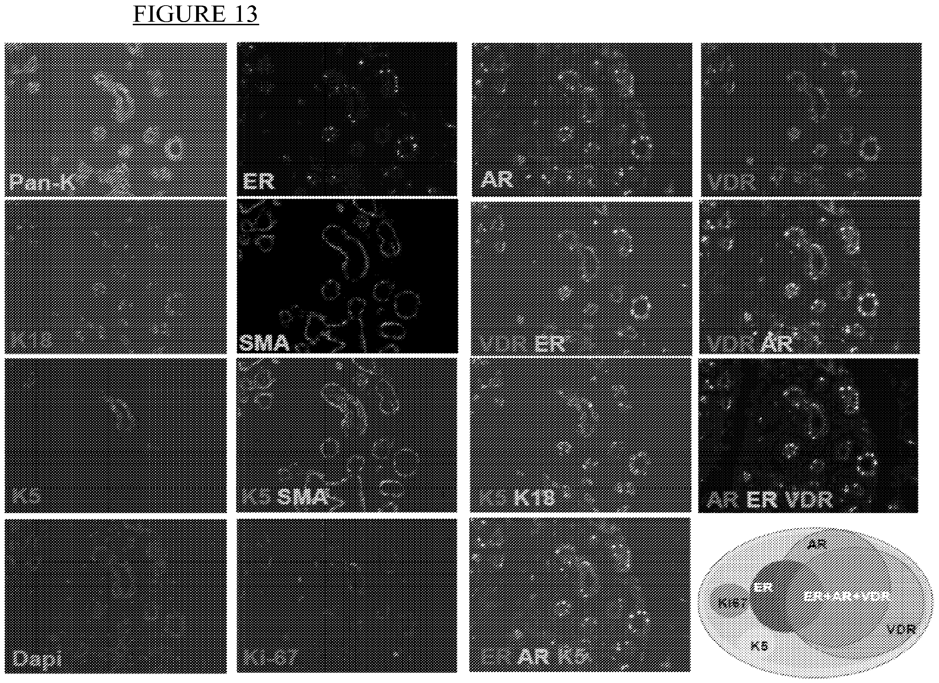

Figure 13 shows multiplex immunofluorescent staining of normal human breast lobules. The same section of normal breast epithelium was evaluated serially for markers Pan-K, K18, K5, ER, SMA, Ki-67, AR, and VDR. Merged images for selected images are shown. A Venn diagram depicting the dynamic relationship of the various markers in this case is shown. This lobule is predominantly composed of AR+ VDR+ and AR/VDR+ cells. The ER+, K5+ and Ki-67+ cells are rare. Representative images from GE Healthcare multiplexed immunofluorescence marker platform are shown.

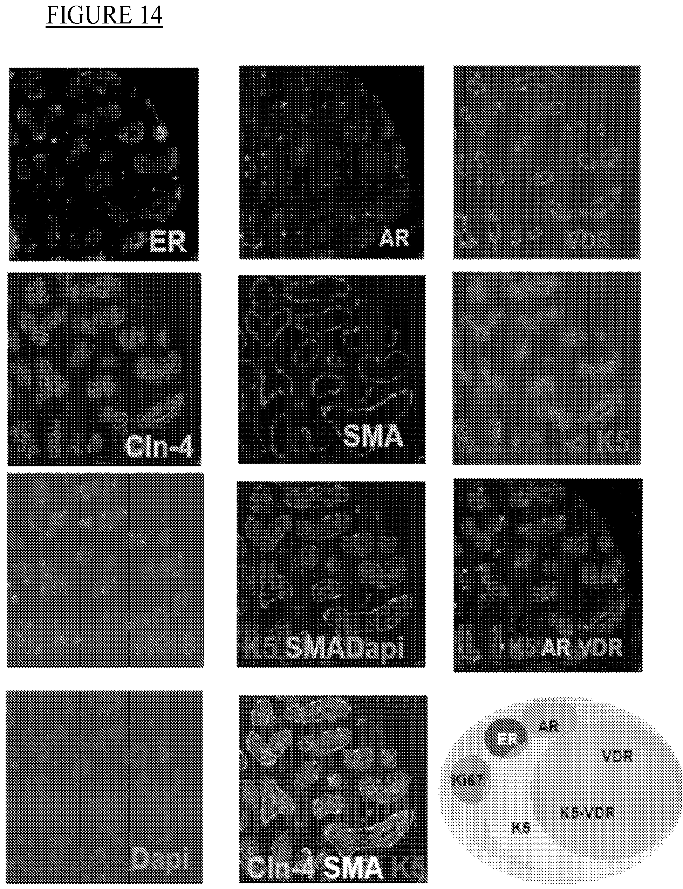

Figure 14 shows multiplex immunofluorescent staining of normal human breast lobules. The same section of normal breast epithelium was evaluated serially for markers ER, AR, VDR, Cln-4 (Claudin-4), SMA, K5, K18. Merged images for selected images are shown. A Venn diagram depicting the dynamic relationship of the various markers in this case is shown. This lobule is predominantly composed of K5+, VDR+ and K5/VDR+ cells. The ER+, AR+ and Ki-67+ cells are rare. Representative images from GE Healthcare multiplexed immunofluorescence marker platform are shown.

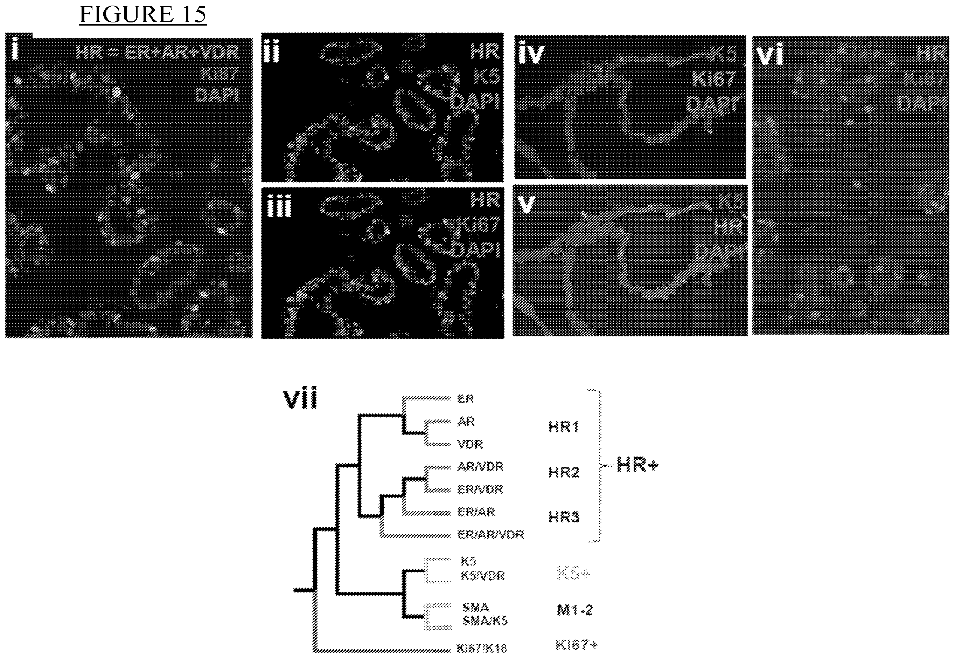

Figure 15 shows merged images of multiplex immunofluorescent staining of the same section of normal human breast lobules. **(i)** Typical breast lobule with moderate levels of hormone receptor (HR) expression and mutually exclusive Ki-67 positive proliferating cells (red). **(ii-iii)** Breast lobule that is almost entirely composed HR+ cells (green) with infrequent K5+ (**ii**) and Ki-67+ (**iii**) cells (red). **(iv-v)** Breast lobule that is almost entirely composed K5+ cells (red), with infrequent Ki-67+ (**iv**) and HR+ (**v**) cells (green). **(vi)** Highly proliferative breast lobule with numerous Ki-67+ cells (red), with no HR+ cells (green). **(vii)** An inferred possible differentiation lineage tree of the breast cell subtypes was constructed using phylogenetic analysis software (Mesquite). According to this model it appears that all the HR+ cells have a common progenitor that is different from the common progenitor of K5+ luminal cells and myoepithelial cells (M1-2). The common progenitor of all three groups (HR+, K5+, and M1-2) appears to be a [Ki-67/K18]-positive and [HR/K5/SMA]-negative cell consistent with a proliferating transit-amplifying progenitor cell type.

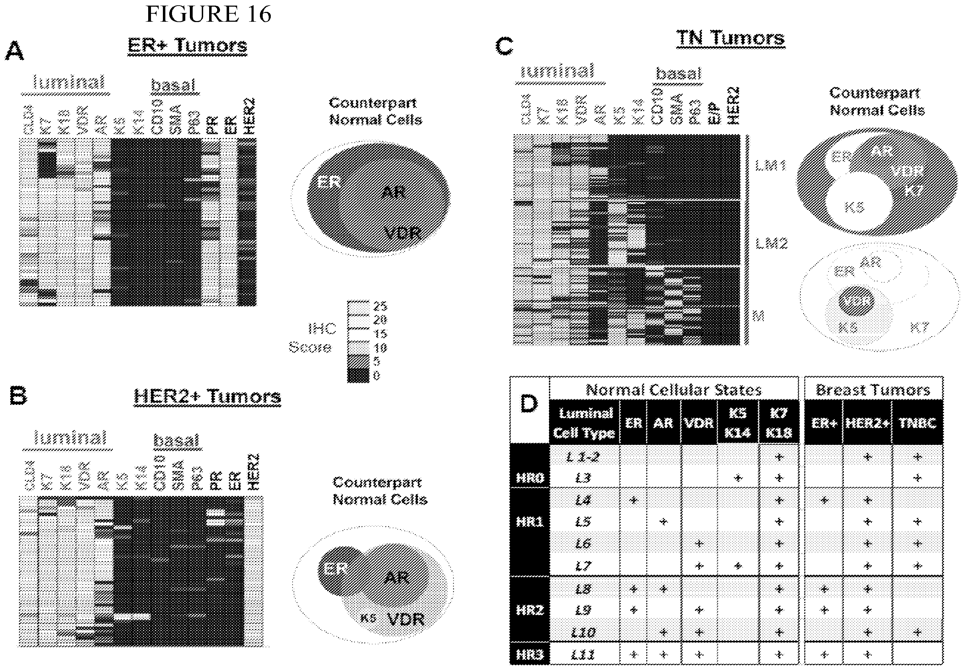

Figure 16 shows identification of normal cellular phenotypes in human breast tumors, as heat maps of protein levels of Cld-4, K7, K18, VDR, AR, K5, K14, CD10, SMA, p63, PR, ER and HER2 in 216 human breast cancer tumors (including 51 ER+, 46 HER2+ and 119 TNBC), separated into **(A)** ER+ (n=51), **(B)** HER2+ (n=46) and **(C)** triple negative breast cancers (n=119). Luminal markers (cld-4, K7, K18, VDR and AR) and basal markers (CD10, SMA and p63) are indicated. Immunostained tissue microarray (TMA) sections were scored using light microscopy and a 0 to 25 scale. The percent of tumor cells staining was quantified as (0) = 0%, (1+) =1-20%, (2+) = 21-40%, (3+) = 41-60%, (4+) = 61-80%, and (5+) = 81-100%. The intensity of staining was quantified 0 to 5. The total score was calculated by multiplying the percent score with the intensity score. Yellow indicates high expression (score 25) and blue indicates low expression (score 0); white is intermediate expression. Venn diagrams of corresponding normal cell counterparts are shown next to the heat maps. Triple negative tumors are separated into LM1 (K5/14-), LM2 (K5/14+) and M (expressing both luminal and myoepithelial markers). Hierarchical clustering performed (pairwise complete linkage). **(D)** Table demonstrating normal cell counterparts corresponding to breast tumor phenotypes observed.

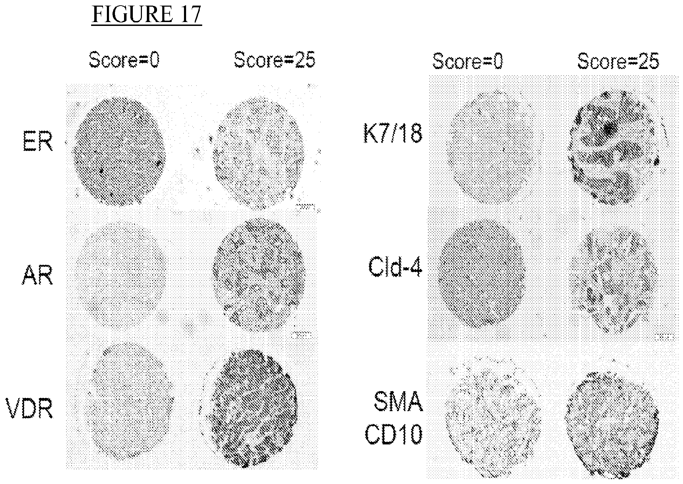

Figure 17 shows expression of cell specific markers in human breast tumors. Similar to normal tissues, ER, AR, VDR, K5, K7, K14, K18, Cld-4, SMA, CD10 were expressed in a binomial pattern in general. A typical example for each marker is shown in adjacent cores in the tissue microarray (TMA) sections; negative cores on the left (<1% staining, and 0 intensity = score of 0) and positives core on the right (>80% staining, and 5 intensity = score of 25). These cores were adjacent to each other on the TMA. One of the advantages of *in situ* staining is the ability to discriminate cell specific expression. For example, SMA/CD10 strongly expressed in the tumor cells in some cases (core on the right with score of 25), and in the stromal cells in other cases (core on the left with a score of 0). This kind of cell type specific expression information is lost in molecular analysis of tumor extracts.

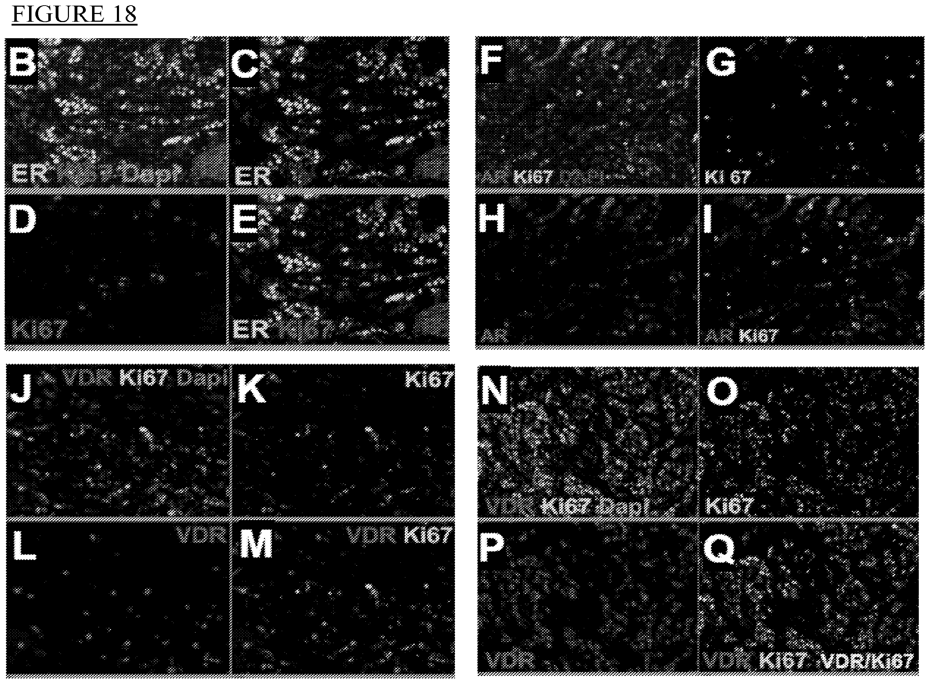

Figure 18 shows expression of cell specific markers in human breast tumors. **(B-E)** Similar to normal tissues, in tumors the proliferating Ki-67[+] tumors cells are mutually exclusive with ER[+] tumor cells. **(F-I)** Similar to normal tissues, in tumors the proliferating Ki-67[+] tumors cells are mutually exclusive with AR[+] tumor cells. **(J-M)** Similar to normal tissues, in tumors the proliferating Ki-67[+] tumors cells are mutually exclusive with VDR[+] tumor cells. **(N-Q)** In some areas the VDR[+] cells were also Ki-67[+], indicating that the relationship between VDR expression and proliferation is different than ER and AR.

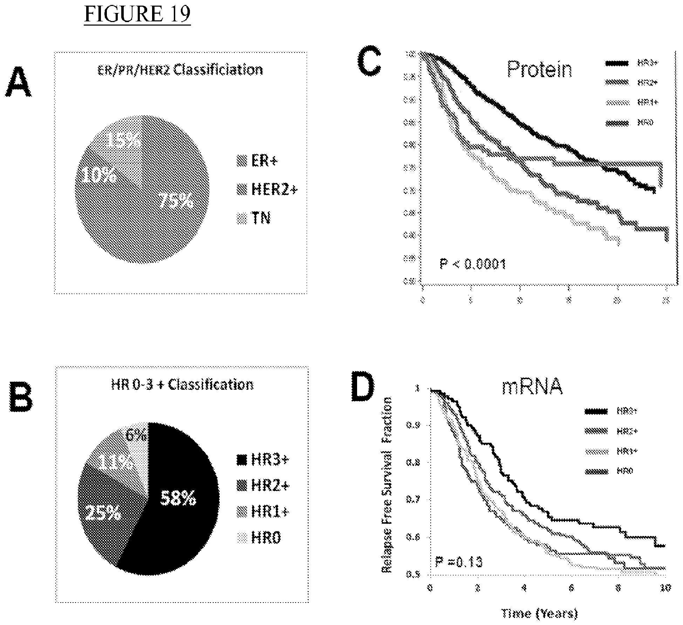

Figure 19 shows different outcomes resulting from normal cell subtype based classification of the instant invention. **(A)** Pie graph demonstrating distribution of ER+, HER2+ and TNBC cases from the full panel of Nurse's Health Study (NHS) cases analyzed in the present studies. **(B)** Reclassification of ER+, HER2+ and TNBC human breast tumors based on HR0-3 categories from the full panel of NHS cases analyzed in this study. Breast tumors were divided in four categories based on normal tissue differentiation: triple-positive tumors (HR3) that co-express ER, AR and VDR, double-positive tumors (HR2) that are ER-AR[+], AR-VDR[+], or ER-VDR[+], single-positive tumors (HR1) that only express one of the hormone receptors (ER[+], VDR[+] or AR[+]), and hormone-receptor negative tumors (HR0) that are negative for ER, AR and VDR. **(C)** Kaplan-Meier analysis for overall survival of all individuals with invasive breast cancer from the Nurses' Health Study that were scored by immunohistochemistry in this study according to hormone receptor status (HR3+ all positive cases, HR2+ double positive case and HR1+ single positive cases). **(D)** Kaplan-Meier analysis of relapse free survival for all invasive breast cancers from an 855 patient breast tumor dataset (37). Tumors were ranked according to gene expression values for ER and scored as 'ER_High' or 'ER_Low' based on a 50% cut off point. The same approach was used to identify 'AR_High and AR_Low' groups, as well as 'VDR_High' or 'VDR_Low'. These groups were then assembled based on HR status HR0, n=141; HR1, n=287; HR2, n=284; HR3, n=143.

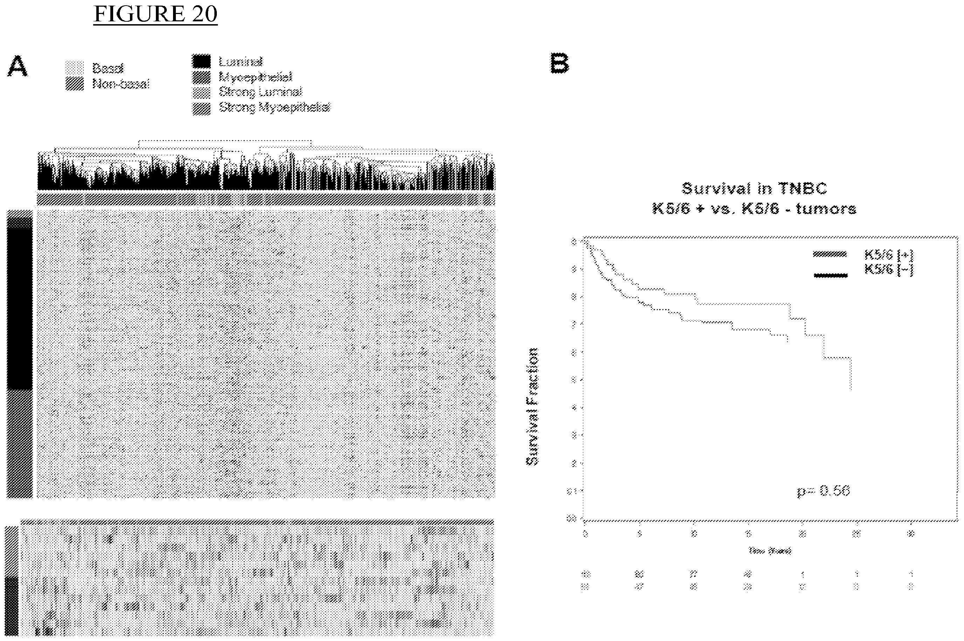

Figure 20 shows expression of keratin 5 and 14 in human breast tumors. **(A)** A**n**alysis of mRNA in normal human breast cells. The normal luminal vs. basal specific profiles were derived by combining three different studies that profiled highly purified luminal vs. myoepithelial cells (6, 25). Only a small subset of mRNAs were differentially expressed between luminal and myoepithelial cells consistently in all three studies. These served as the basis for strong luminal (green bar) and strong myoepithelial (dark blue) consensus signatures. Differential expression in two of three studies formed the basis for luminal (black) and myoepithelial (red) signatures, depicted with the colored bars on the left hand side of the heatmap (29-31, 54). When a combined human breast mRNA expression dataset was analyzed for the expression of these luminal basal genes, there was no significant difference between basal tumors vs. luminal (non-basal) tumors , marked with colored bars above the heatmap; basal tumors (light blue) vs. luminal (non-basal) tumors (pink). Rows indicate genes, columns indicate tumor sample from each patient; over-expression shown in orange, under-expression shown in blue. **(B)** Kaplan-Meier analysis of all individuals with triple negative invasive breast cancer from the Nurses' Health Study that were scored by K5/6 immunohistochemistry (n=172). There was no statistically significant survival difference between the K5/6[+] (n=59) vs. K5/6[-] (n=113) tumors (p=0.56).

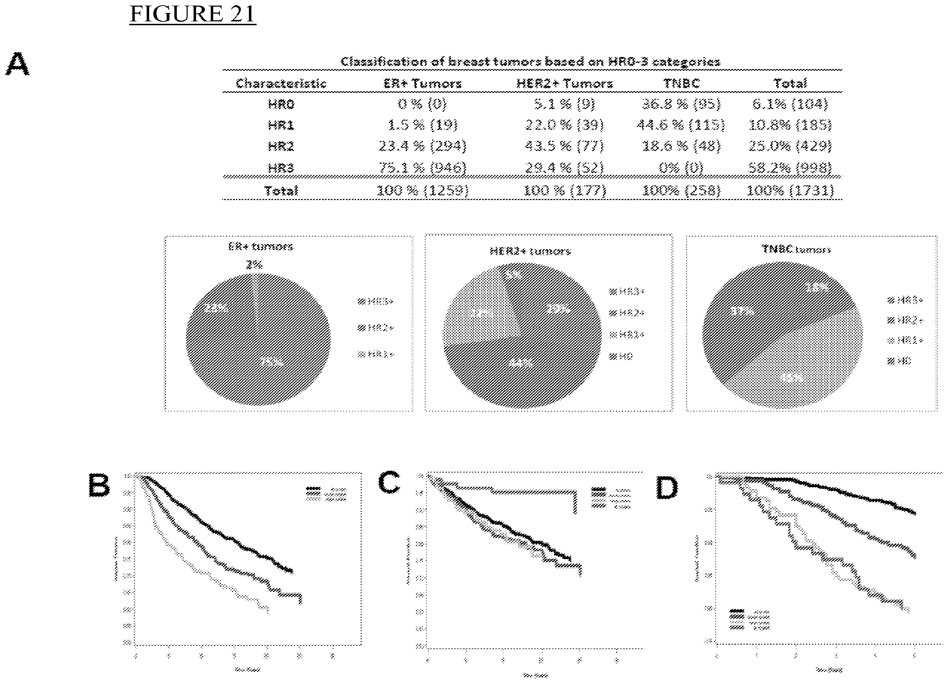

Figure 21 shows reclassification of human breast tumors based HR0-3 categories and drug response of breast cancer cell lines. **(A)** Table showing the frequency of HR 0, 1,2 and 3 tumors in the clinical categories of ER+, HER2+ and TNBC. **(B)** Venn diagrams showing the composition of breast tumors based on HR classification. **(C-D)** Kaplan-Meier analysis of all individuals with invasive breast cancer from the Nurses' Health Study that were scored by immunohistochemistry in this study according to hormone receptor categories (HR3+ = black curve, HR2+ = red curve, HR1+ = yellow curve, HR0 = blue curve). Survival was examined as a continuous variable during 0-5 years (C) and during 5-25 years (D).

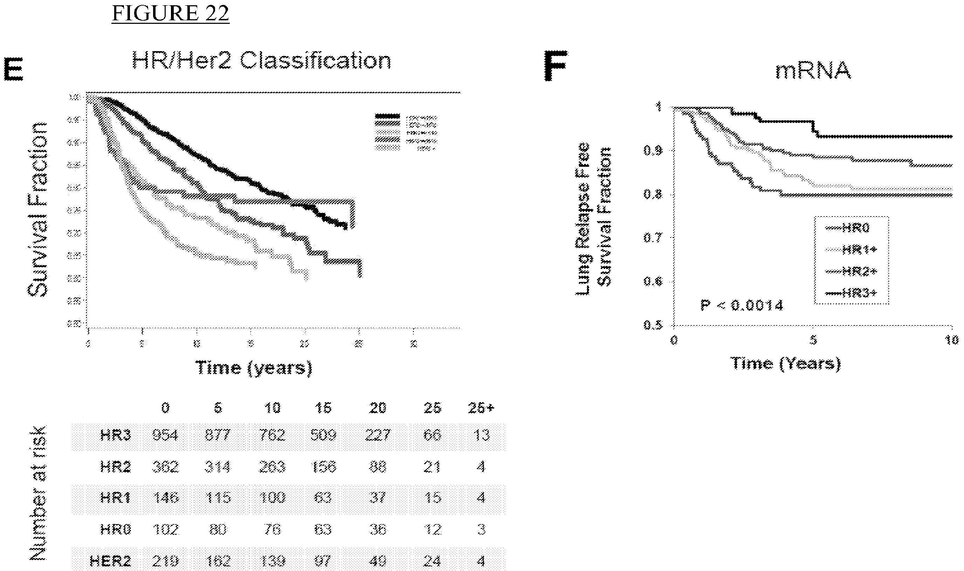

Figure 22 shows reclassification of human breast tumors based HR0-3 categories and drug response of breast cancer cell lines. **(E)** Kaplan-Meier analysis of all individuals with invasive breast cancer from the Nurses' Health Study. In this analysis the patients with HER2+ tumors were analyzed as a distinct group. (HR3+ = black curve, HR2+ = red curve, HR1+ = yellow curve, HR0 = blue curve, HER2+ = pink curve). (**F**) Kaplan-Meier analysis of lung metastasis free survival for all invasive breast cancers from an 855 patient breast tumor dataset (37). Tumors were ranked according to gene expression values for ER and scored as 'ER_High' or 'ER_Low' based on a 50% cutpoint. The same approach was used to identify 'AR_High and AR_Low' groups, as well as 'VDR_High' or 'VDR_Low'. These groups were then assembled based on HR status

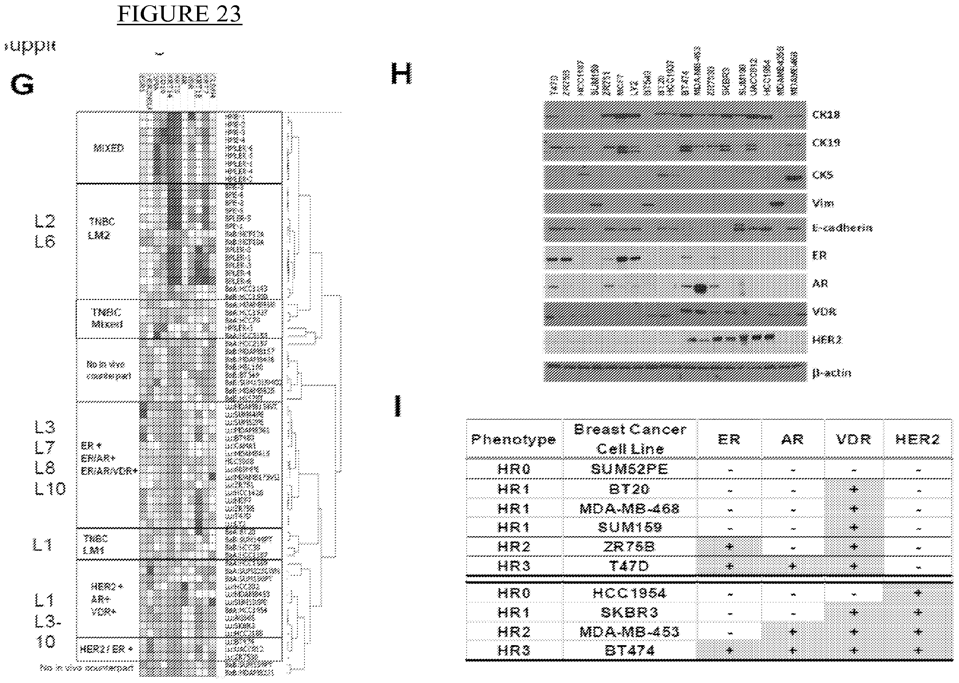

Figure 23 shows reclassification of human breast tumors based HR0-3 categories and drug response of breast cancer cell lines. **(G)** HR0-3 classification of human breast cancer cell lines based on mRNA expression. Breast cancer cell lines were clustered by mRNA expression of differentiation state transcripts (Cld-4, K7, K18, VDR, AR, K5, K14, CD10, SMA, HER2, ESR1 (ER). Cell lines were grouped according to the normal differentiation state that they represent (L1-L11). Clustering was performed as described in the Materials and Methods section below. Red indicates a relative increase in expression, green indicates a relative reduction in expression and white indicates no change. **(H, I)** Western blot analysis of markers in breast cancer cells lines and summary table of the results.

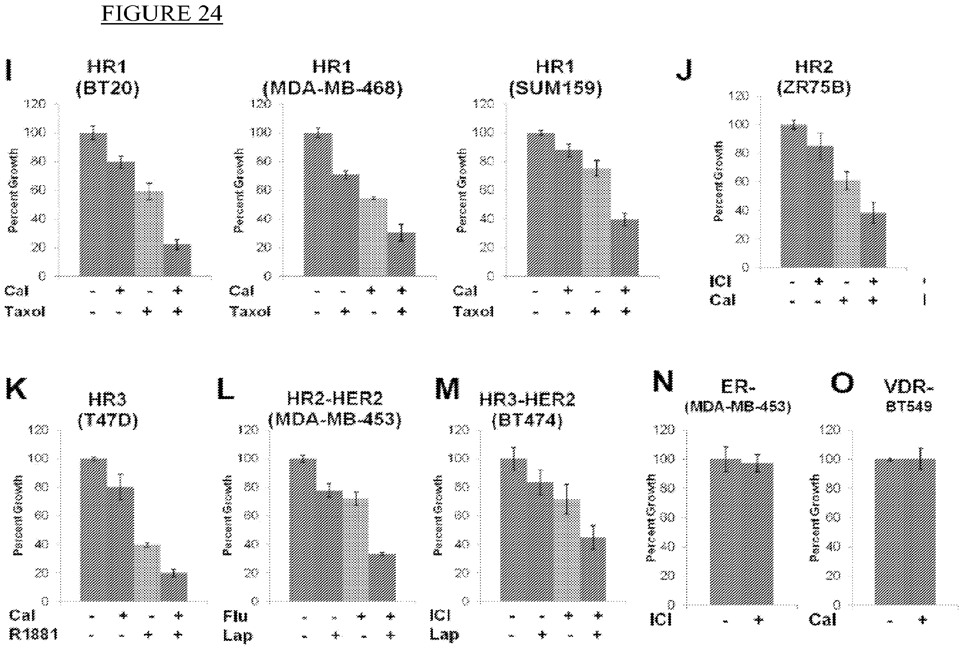

Figure 24 shows reclassification of human breast tumors based HR0-3 categories and drug response of breast cancer cell lines. **(J-P)** Response of HR1-3 breast cancer cell lines to combined hormone therapy. In all experiments described below (J-P) low doses of individual drugs (< 50% inhibition) were used in order to demonstrate the additive effect of two drugs combined. Each experiment was repeated multiple times with similar results; representative results from one experiment is shown. **(J)** VDR and Taxol combination treatment of HR1 cell lines: The ER/HER2 negative breast cancer cell lines BT20, MDA-MB-468 and SUM159 are traditionally considered TNBC models, hence not candidates for hormone treatment. However, according to our HR classification these HR1 (VDR+) tumors can be candidates for treatment with VDR agonists. Consistent with this, a reduction in proliferation was observed when these HR1 breast cancer cells were treated with VDR agonist Calcitrol (Cal, 25nM) alone or in combination with a chemotherapeutic such as Taxol (0.5nM or 1nM). The combined effect of these drugs was greater than either drug alone, indicating an additive effect. In the clinical setting this may allow using less toxic doses of each drug with the same efficacy as using higher doses of Taxol alone. These results indicate that combining VDR agonists with chemotherapy should be explored further for in HR1 subtype of TNBC in combination with chemotherapy. **(K)** ER and VDR combination treatment of HR2 cell lines: The breast cancer cell line ZR75B is a model of ER/VDR+ HR2 tumors that could be potentially targeted with ER/VDR combination hormone therapy. The ER-antagonist ICI 182,780 (Faslodex, ICI 5nM) was combined with VDR agonist Calcitrol (Cal, 50nM), resulting in a combined effect of these drugs that was greater than using either drug alone. These results indicate that combining ER antagonists with VDR agonists should be explored further in this subtype of HR2 breast cancers. **(L)** AR and VDR combination treatment of HR3 cell lines: The breast cancer cell line ER+ T47D expresses AR and VDR (HR3). In this cell line combination of AR-agonist R1881 (Methyltrienolone, 50nM) with VDR agonist Calcitrol (Cal, 50nM) inhibited proliferation more effectively than either drug alone. These experiments were carried out in phenol red free DMEM +5% charcoal stripped FBS and 17-beta estradiol (E2, 10nM) as previously described (55, 56). Representative results from multiple experiments are shown. These results indicate that combining AR and VDR agonists should be explored further in this subtype of HR3 breast cancers. **(M)** AR and HER2 combination treatment of HR2/HER2 cell lines: The combination of AR-antagonist Flutamide (Flu 45 µM) and HER2 inhibitor Lapatnib (Lap, 0.5µM) additively inhibited proliferation HR2+/HER2+ breast cancer cell line MDA-MB-453. **(N)** ER and HER2 combination treatment of HR2/HER2 cell lines: The combination ER-antagonist ICI 182,780 (Faslodex, ICI 10nM) and HER2 antagonist Lapatnib (Lap, 10nM) additively inhibited proliferation HR3+/HER2+ breast cancer cell line BT474, as previously described (57). Representative results from multiple experiments are shown. The above results **(M, N)** indicate that combining ER and AR antagonists with HER2 targeted therapy should be explored further for HER2+/HR+ breast cancers. **(O)** In ER-negative control cell line (MDA-MB-453) no inhibition of cell proliferation was seen when it was treated with ER antagonist ICI 182,780 even at a much higher concentration (Faslodex, ICI 100nM). **(P)** In VDR-negative control cell line (BT549) no inhibition of cell proliferation was seen when it was treated with VDR agonist Calcitriol (Cal) at different concentrations of 10nM, 25nM and 100nM. Control and 100nM groups are also shown.

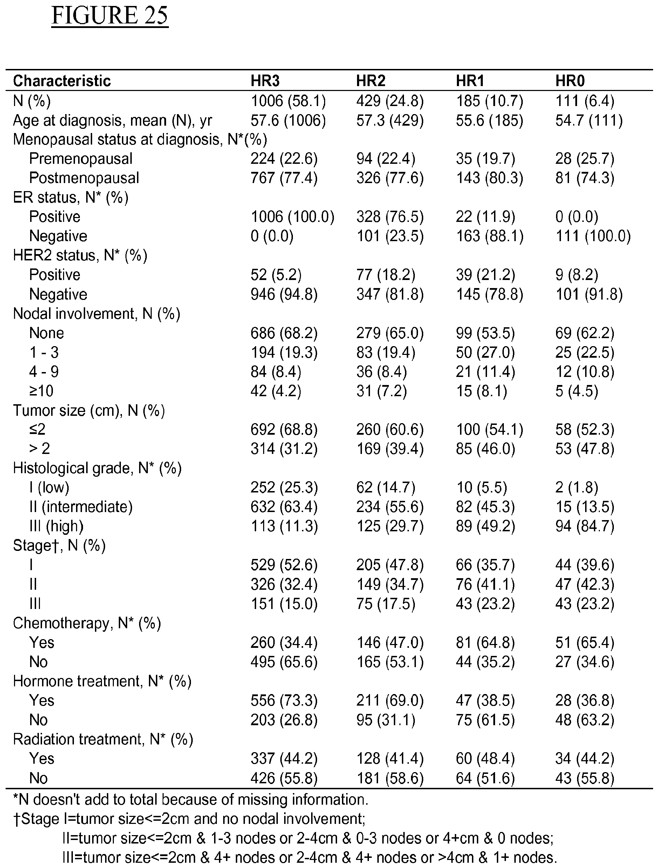

Figure 25 is a table showing the means and frequencies of participants' characteristics by cross-classified ER/AR/VDR status (N=1731) in the Nurses' Health Study (1976-1996).

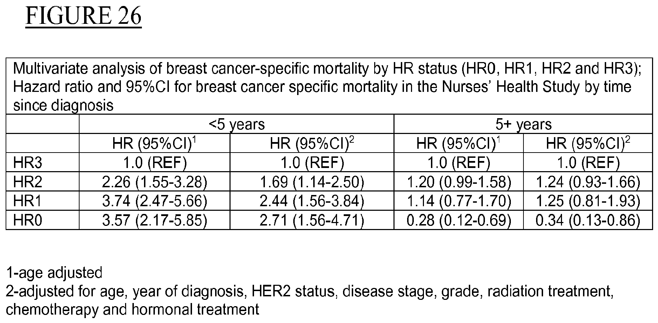

Figure 26 is a table showing the multivariate analysis of breast cancer-specific mortality by HR status (HR0, HR1, HR2 and HR3), hazard ratio and 95%CI for breast cancer specific mortality in the Nurses' Health Study by time since diagnosis.

Like reference numerals refer to like parts throughout the several views of the drawings.

### DETAILED DESCRIPTION

The present invention is directed to a novel classification system for breast cancer, and methods of treatment based on such classification system. This system is based on expression levels of certain hormone receptors in the breast tissue, which mirror the phenotypic cellular expression of these same hormone receptors in normal non-cancerous breast tissue. The present invention relates currently to eleven cell subtypes of breast cancer conform to four particular hormonal differentiation groups, denoted herein as HR0-3. Moreover, each differentiation group correlates with varying degrees of survival success. This ontological classification system therefore also provides actionable hormone treatment strategies for all subtypes of human breast cancer, which may be personalized for the patient and the particular hormonal expression pattern they exhibit.

It should be appreciated that the classification system and methods of diagnosing, predicting prognosis, and treating based on the classification system of the present invention applies equally to any cancers in which hormones play a role. For example, even though the present invention is described and explained herein in terms of breast cancer, it applies to ovarian, prostate, and thyroid cancers as well, to name but a few for illustration purposes.

Prior to setting forth the invention in detail, it may be helpful to the understanding thereof to define several terms, and these are accordingly set forth in the next section, below. Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. It will be further understood that terms, such as those defined in commonly used dictionaries, should be interpreted as having a meaning that is consistent with their meaning in the context of the relevant art and/or as otherwise defined herein.

As used herein, the term "patient" refers to any animal, such as but not limited to humans, which is to be the recipient of a particular treatment. Other non-limiting examples of animals which may be a patient includes mammals, birds, reptiles, amphibians, fish, non-human primates, rodents, and the like. Typically, the terms "patient" and "subject" may be used interchangeably herein.

As used herein, the terms "expressing" or "expression" refer to the levels of a protein, mRNA, DNA, gene or gene fragment, biological and/or biochemical activity of a particular nucleotide sequence, gene or gene fragment. The terms "expressing" and "expression" may be used interchangeably.

Furthermore, a significant or relevant level of expression means expression of the protein, mRNA, DNA, gene or gene fragment, or biological and/or biochemical activity or pathway to such a degree that expression level correlates with disease state and/or progression and/or remission thereof or correlates with response to ligands (such as agonists and antagonists) that inhibit or activate said protein, mRNA, DNA, gene or gene fragment, or biological and/or biochemical activity. The response to ligands may be any appropriate biological, chemical, biochemical, medical, pathological, phenotypic, and/or any other response to ligand-receptor binding as may be observed and/or measured *in vitro* or *in vivo.* Illustrative examples include, but are not limited to, increase or decrease in cell proliferation and/or cell growth, apoptosis, differentiation, growth arrest, migration, invasion, increase or decrease in metastasis, tumor size, disease-free survival, recurrence-free survival, overall survival, etc.

Levels of expression falling short of biological and/or biochemical relevance are considered to be not expressed or substantially lacking expression. Expression may be qualitative or quantitative, and may be quantified discretely or continuously, and certain thresholds may be set anywhere along the continuum to signify various levels of expression. For example, expression may be determined by the number of cells in a population exhibiting any detectable amount of protein, mRNA, DNA, gene or gene fragment, biological and/or biochemical activity of a particular nucleotide sequence, gene or gene fragment. In other embodiments, expression may be determined by the level, degree, or intensity of the detectable amount of protein, mRNA, DNA, gene or gene fragment, biological and/or biochemical activity of a particular nucleotide sequence, gene or gene fragment. Expression may also be determined by a combination of quantitative and qualitative characteristics.

As used herein, "disease" refers to a disorder, disease or condition, or other departure from healthy or normal biological activity which impairs normal function. The terms "disease", "disorder", and "condition" may be used interchangeably throughout the present specification. The condition may be caused by sporadic or heritable genetic abnormalities. The condition may also be caused by non-genetic abnormalities. The disease may be present in a subject with clinical or sub-clinical symptoms; however, the subject may also be asymptomatic at the time of diagnosis. The disease may include a benign growth or benign tumor (i.e., non-cancerous growth), as well as cancer (i.e., cancerous cell or cancerous tumor). For instance, at least one embodiment "disease" as used herein refers to breast cancer, and more specifically human breast cancer.

The term "cancer" or "cancerous," as used herein, refers to the uncontrolled growth and division of cells, resulting in an overgrowth of cells, or mass or growth (i.e., tumor). It includes both benign and metastatic forms of cancer. When referring to a "tumor," a cancerous tumor is often referred to as a "malignant tumor"; however, reference to a "tumor" alone may also refer to a malignant tumor in some contexts. "Cancer" collectively refers to individual cancerous cells and tumors, such as more than one, or a mass, of cancerous cells. Additionally, references to "cancer" include primary tumors that grow at the site of tumor origination and proceed to form a cancerous growth, as well as and metastatic lesions or one or more tumors that are derived from a malignant cell(s) of the primary tumor but grow at a site remote from the primary tumor. However, the use of the term "cancer" does not necessarily mean that a primary tumor has yet metastasized. Some cancers may be either indolent or aggressive initially. In contrast, some cancers may begin as an indolent lesion and change over time to become aggressive. As such, a primary tumor may remain indolent for a period of time and grow very slowly with no metastatic occurrence and eventually become aggressive and grow rapidly and metastasize. Use of the terms "cancer" and "tumor" would encompass all such possibilities described above as utilized in the present invention.

As used herein, "treatment" or "treating" refers to arresting or inhibiting, or attempting to arrest or inhibit, the development or progression of a disorder and/or causing, or attempting to cause, the reduction, suppression, regression, or remission of a disorder, disease and/or a symptom thereof. As would be understood by those skilled in the art, various clinical and scientific methodologies and assays may be used to assess the development or progression of a disease, and similarly, various clinical and scientific methodologies and assays may be used to assess the reduction, regression, or remission of a disease or its symptoms. "Treatment" refers to both therapeutic treatment and prophylactic or preventative measures. Those in need of treatment include those already with the disease as well as those prone to have the disease or those in whom the disease is to be prevented.

As used herein, the term "administering" refers to providing a therapeutically effective amount of a treatment product to a subject. The treatment product is contemplated as a chemical, biochemical or pharmaceutical molecule or compound capable of impacting the signaling pathway for at least one of the hormones involved in the classification system herein, including estrogen receptor (ER), androgen receptor (AR), vitamin D receptor (VDR), and HER2. For instance, the treatment product may be an ER antagonist, such as but not limited to ICI 182, 780 (Faslodex). The treatment product may be an AR agonist such as R1881 (Methyltrienolone) or a VDR agonist such as Calcitrol as non-limiting examples. The treatment product may directly affect the hormone receptor, or may work by affecting "partner proteins" that interact with or have an effect on hormones and/or hormone receptors for signaling and downstream effects. Moreover, the treatment product may be administered or provided to the patient using any appropriate method of administration, such as but not limited to oral, intravitreal, intraocular, ocular, subretinal, intrathecal, intravenous, subcutaneous, transcutaneous, intracutaneous, intracranial, and topical. The treatment product can be administered alone or with other compounds, excipients, fillers, binders, carriers or other vehicles selected based upon the chosen route of administration and standard pharmaceutical practice. Administration may be by way of carriers or vehicles, such as injectable solutions, including sterile aqueous or non-aqueous solutions, or saline solutions; creams; lotions; capsules; tablets; granules; pellets; powders; suspensions, emulsions, or microemulsions; patches; micelles; liposomes; vesicles; implants, including microimplants; eye drops; other proteins and peptides; synthetic polymers; microspheres; nanoparticles; and the like.

The treatment product may also be included, or packaged, with other non-toxic compounds, such as pharmaceutically acceptable carriers, excipients, binders and fillers including, but not limited to, glucose, lactose, gum acacia, gelatin, mannitol, xanthan gum, locust bean gum, galactose, oligosaccharides and/or polysaccharides, starch paste, magnesium trisilicate, talc, corn starch, starch fragments, keratin, colloidal silica, potato starch, urea, dextrans, dextrins, and the like. Specifically, the pharmaceutically acceptable carriers, excipients, binders, and fillers contemplated for use in the practice of the present invention are those which render the compounds of the invention amenable to oral delivery, intravitreal delivery, intraocular delivery, ocular delivery, subretinal delivery, intrathecal delivery, intravenous delivery, subcutaneous delivery, transcutaneous delivery, intracutaneous delivery, intracranial delivery, topical delivery and the like. Moreover, the packaging material may be biologically inert or lack bioactivity, such as plastic polymers, silicone, etc., and may be processed internally by the patient without affecting the effectiveness of the treatment product packaged and/or delivered therewith.

The term "effective amount" or "therapeutically effective amount", which may be used interchangeably, as applied to the treatment products described herein means the quantity necessary to render the desired therapeutic result. For example, an effective amount is a level effective to treat, cure, or alleviate the symptoms of a disease for which the therapeutic compound, biologic or composition is being administered, such as cancer and more specifically breast cancer. Amounts effective for the particular therapeutic goal sought will depend upon a variety of factors including the disease being treated and its severity and/or stage of development/progression; the bioavailability, and activity of the specific compound, biologic or pharmaceutical composition used; the route or method of administration and introduction site on the subject; the rate of clearance of the specific compound or biologic and other pharmacokinetic properties; the duration of treatment; inoculation regimen; drugs used in combination or coincident with the specific compound, biologic or composition; the age, body weight, sex, diet, physiology and general health of the subject being treated; and like factors well known to one of skill in the relevant scientific art. Some variation in dosage will necessarily occur depending upon the condition of the subject being treated, and the physician or other individual administering treatment will, in any event, determine the appropriate dose for an individual patient.

The term "antagonist" as used herein means a molecule, pharmaceutical, drug, or compound that binds a receptor and thereby blocks the ability of other molecules to bind the same receptor. Antagonists do not produce a biological response upon binding its target receptor, and yet their binding prevents the binding of other molecules which could cause a biological effect. Antagonists are therefore considered inhibitory molecules that inhibit or block downstream events in a biochemical pathway in a living organism. The effect of an antagonist may be a result of binding to an active site on a receptor, which is the same site as another molecule would bind the same receptor. The antagonist effect may also be a result of allosteric binding, in which the antagonist binds a different site on the receptor than another molecule would bind, but which nevertheless prevents the binding of that other molecule. The antagonist may have varying levels of affinity for the receptor binding site, and may therefore compete and/or outcompete other endogenous ligands or substrates for binding with the receptor. Further, the antagonist-receptor binding may be reversible or irreversible depending on the antagonist, receptor, binding site properties, and affinities thereof.

The term "agonist" as used herein means a molecule, pharmaceutical, drug, or compound that binds a receptor so as to trigger a response. For instance, the receptor may be expressed on or within a cell, and the binding of an agonist will cause an effect or change on or within the cell. The effect can be biological or biochemical in nature. For instance, the agonist binding to the receptor may induce the expression of a particular protein, or may cause the activation of a biochemical pathway or "cascade" of reactions in a biochemical pathway. The agonist may be selective for a particular receptor or binding site on a receptor, or may be general to a class of receptors or binding sites. Further the agonist may bind with varying affinity and specificity for its target binding site, depending on the particular agonist and target receptor. The agonist may be competitive with other ligands that bind similar receptors and/or binding sites, or may be unique to a receptor and/or binding site. Further, the agonist-receptor binding may be reversible or irreversible depending on the agonist, receptor, binding site properties, and affinities thereof.

With these definitions in mind, we now turn to the present invention. Specifically, the present invention is directed to a classification system for breast cancer based on normal breast cell phenotypes of expression of certain proteins, namely, estrogen receptor (ER), androgen receptor (AR), and vitamin D receptor (VDR), and in some cases HER2, as well as certain markers such as keratins that are differentially expressed in the luminal layer and myoepithelial layer of breast tissue.

The classification system is based on the presence of these particular receptors and markers for a few reasons. First, an initial review of literature and preliminary immunostains was conducted for hormone receptors, as these are principally responsible for tissue growth and differentiation. The initial review revealed three receptors ER, AR, and VDR stood out with distinct bi-modal expression patterns in the luminal layer of breast lobules, being strongly expressed in some cells and not expressed at all in others. Many other hormone receptors did not exhibit bi-modal expression, and therefore were not useful as markers to identify cellular subtypes (i.e. luminal vs. myoepithelial) in the breast tissue. In addition, certain proteins and keratins, such as K7, K8, K18, K5, Ki67 (marker for proliferating cells), and Claudin-4 (Cld-4) are differentially expressed in the luminal layer cells of breast tissue. Other keratins and proteins, including cluster of differentiation marker CD10, smooth muscle actin (SMA) and p63 are localized to the myoepithelial layer cells of breast tissue. The luminal layer contains proliferating or growing cells, and since cancer is essentially uncontrolled growth of cells, understanding the phenotypes of cells in the luminal layer of breast tissue was considered to be an important step in understanding and classifying breast cancer. Moreover, 95% of human tumors are similar in phenotype to luminal layer cells, and almost all the proliferation in normal breast takes place in the luminal layer.

It should be noted that the present invention includes classification and methods based on any form of ER, AR, and VDR, and any of the ligands which they will bind. For example, the present invention can be used to identify activity based on steroidal and non-steroidal estrogens, such as but not limited to estrone, estradiol, estriol, sterol, and xenoestrogens. Similarly, it can be used to identify activity based on any androgens with AR, such as adrenal adrogens, and may include but are not limited to testosterone, dihydrotestosterone (DHT), dehydroepiandrosterone (DHEA), androstenedione, androstenediol, and androsterone. In addition, any vitamin D may be implicated in VDR expression involved in the present invention, such as but not limited to vitamin D2 and D3.

More in particular, the classification system of the present invention includes a first category defined by a substantial lack of expression of any of ER, AR, and VDR in a breast cancer tumor or cancerous tissue. Therefore, this category may also be referred to herein as a hormone receptor negative state, or HR0, although any name may be used which corresponds to a lack of expression of these three receptors. This naming strategy applies similarly to the other categories explained below.

In order to fall into the HR0 category, the breast tissue must not express significant or relevant levels of ER, AR, or VDR, although it may express other proteins. As used herein, a "substantial lack" of expression means levels of expression that are not functionally relevant, such as will not generate downstream effects for example. Accordingly, it is feasible that some small level of ER, AR, and/or VDR may be present and/or detectable in this category, and yet be present in such small quantities as to be negligible. By way of example, and not to be construed in a limiting sense, in at least one example a substantial lack of expression of ER, AR, and/or VDR may be determined when there are less than 1% of cells in a tissue sample shown to express ER, AR, and/or VDR. In other embodiments, the threshold for substantial lack of expression of ER, AR, and/or VDR is less than 5%, less than 10%, less than 30%, less than 50%, and anywhere in a continuous range between 0% and 50% of cells expressing. In still other embodiments, expression may be determined not by the number of cells expressing ER, AR, and/or VDR, but rather by the intensity or degree of expression. For instance, intensities of less than 1 or less than 50% may be considered to be not functionally or significantly relevant. Intensity too may be considered on a continuous or discrete scale for expression determinations.

Moreover, this first category (HR0) includes a number of subtypes, or subclasses, denoted as L1-L3 in Figure 10. While all of these subtypes are classified as negative for ER, AR, and VDR, some breast tissues express the cell proliferation marker Ki67 (L1), while others express the keratin K8 (L2) and still other express keratin K5 (L3).

The classification system also includes a second category which is defined by expression of any one of ER, AR, or VDR. Therefore, this category may also be referred to herein as a single hormone receptor positive state, or HR1 or HR1+. Any of the receptors ER, AR, or VDR may be present in order to fall into this category, but only one can be expressed. As defined previously, ER, AR, and/or VDR are considered to be expressed when they are present in levels rising to functionally significant and/or relevant. In at least one embodiment, expression may be determined by at least 1% of cells exhibiting ER, AR, and/or VDR in any form, such as protein, mRNA, DNA, or bioactivity. In other embodiments, the threshold for expression is defined as at least 5%, at least 10%, at least 30%, and at least 50%. As before, expression may be defined qualitatively or quantitatively, and may be discrete or along a continuum.

This expression is in the luminal layer of breast tissue. Accordingly, and as seen in Figure 10, there are a number of subtypes or subclasses of HR1 (L4-L7). For instance, one subtype corresponds to those tissues expressing only ER (L4). Another subtype corresponds to tissues expressing only AR (L5). Another subtype corresponds to tissues expressing only VDR (L6). A final subtype corresponds to tissues expressing only VDR, but which also express the keratin K5 (L7).

The classification system includes a third category which is defined by the expression of a combination of any two of ER, AR, and VDR. Therefore, this category may also be referred to herein as a double hormone receptor positive state, or HR2 or HR2+. As before, this expression is in the luminal layer. Accordingly, there are three subtypes, denoted as L8-L10 in Figure 10, corresponding to each particular combination. For example, L8 corresponds to cells expressing both ER and AR. L9 corresponds to cells expressing both ER and VDR. L10 corresponds to cells expressing both AR and VDR.

The classification system also includes a fourth category which is defined by the expression of all three of ER, AR, and VDR. Therefore, this category may also be referred to herein as a triple hormone receptor positive state, or HR3 or HR3+. This is also considered to be its own subclass L11, as seen in Figure 10.

In certain embodiments, the classification system also encompasses the expression of human epidermal growth factor receptor 2 (HER2), which is known to be associated with some types of breast cancers. Its expression has typically been considered an indication of more aggressive breast cancer, and is considered to be more resistant to hormone treatment.

This classification system of the present invention is used to classify breast cancer tumors or cancerous tissue, be they benign or malignant. It is based on similar phenotypic expression patterns of ER, AR, and VDR in normal breast tissue, which is unique in a number of ways. First, the present system is a departure from known breast cancer classification systems in its comparison to normal tissue for a phenotypic characterization. This allows a more accurate identification of subtypes of cancer. Second, it is the only classification system that evaluates AR or VDR levels in determining tumor classification, as compared to known classification systems that look only at ER, HER2 and triple-negative tumors for identification. (See Figure 19 and Example 9 below for more detail). Indeed, the present invention is an integrated system taking into consideration ER, AR, and VDR, and in some cases HER2 expression levels, taken together. These aspects of the invention demonstrate the present classification system is not merely a renaming of existing groups, but provides a better organization of tumor classification than currently exists, which permits more accurate diagnoses and the design of more effective treatment regimens.

The present invention is also directed to a method of classifying breast cancer utilizing the instant classification system. Essentially, the method includes measuring the levels of ER, AR, and VDR present in a breast cancer tumor or cancerous tissue of a patient, and classifying the cancer based on the expression levels of these proteins. In some embodiments, it also includes measuring levels of HER2 and classifying the cancer based on levels of ER, AR, VDR, and HER2 present. "Measuring" levels as used throughout this specification can be performed by any appropriate chemical, biological, and/or biochemical method as may be understood to those of skill in the art. Examples include, but are not limited to: antibody based assays such as immunostaining, immunofluorescence, immunoassays, and immunoprecipitation; mass spectroscopy of protein, DNA, or RNA; protein studies such as Western blot analysis; nucleic acid studies such as Northern blot analysis, probe hybridization, mRNA studies, polymerase chain reactions (PCR) of DNA or RNA, in situ hybridizations; and bioassays to detect biological and/or biochemical activity. It is contemplated within the present method that the levels of ER, AR, and/or VDR measured can include any value including zero or substantially or functionally zero as previously described, and may be discrete or continuous. Such information is utilized in classifying the cancer into categories. As explained in greater detail previously, as well as in the Materials and Methods section, ER, AR, and VDR positive status can be assigned when greater than 1% of cells in the sample show any expression or activity of the above. In other embodiments, positive status is defined as greater than 5% of cells, greater than 10% of cells, and/or greater than 50% of cells. Various increments in between are also included in the spirit of the invention.

More in particular, in at least one embodiment the method includes classifying the cancer into one of a plurality of categories based on the levels of expression of ER, AR, and VDR measured in the tumor or tissue sample. These categories are as previously described in the explanation of the classification system of the present invention, and include a first category (i.e. HR0), second category (HR1), third category (HR2) and fourth category (HR3). The present method therefore also includes comparing the levels of ER, AR, and VDR measured in the tumor or cancerous tissue to similar expression patterns and/or levels of ER, AR, and VDR in normal corresponding tissue, such as breast tissue. It should be appreciated that "tissue" as used herein includes at least one cell, as tissues are comprised of a community of similar type cells, such as of the same origin.

In addition, the various categories of the instant classification system (denoted as HR0-HR3+ in the Figures and Examples provided below) correspond to significant survival differences in patients diagnosed with breast cancer. As used herein, "significant" means statistically significant such that the event is most likely not caused by chance, but rather the direct result of a particular cause - in this case, the presence of ER, AR, and/or VDR and combinations thereof. For instance, as shown in Figure 19 and explained in greater detail in Example 10 below, patients with tumors classified into the fourth category (i.e., HR3) which express all of ER, AR, and VDR have the best survival rates (and therefore better prognosis) as compared to patients with tumors classified into other categories. Third category (i.e., HR2) tumors expressing any two of ER, AR, and VDR do less well, but tumors in the second category (i.e., HR1) that express only one of ER, AR, or VDR have the worst survival rate, and therefore prognosis, compared to other categories. This is visually represented in Figure 19, in which Kaplan-Meier curves show that more patients with tumors classified as HR3+ are alive at any given time point (i.e., number of years) after diagnosis as compared to patients having HR2+ tumors, which fare better than patients having HR1+ tumors. As used herein, category designations used with and without a "+" are equivalent and may be used interchangeably (e.g., HR3 and HR3+). With HR0 tumors, the chances of survival level out beyond five years after diagnosis and do better over time than the other tumors, but initially do worse than other tumors. Based on this information, resulting from the classification system of the present invention, more accurate prognoses can be provided to patients.

Accordingly, the present invention is further directed to a method of predicting a prognosis of breast cancer, which includes measuring the levels of ER, AR, and VDR present in a tumor or cancerous tissue of a patient. Based on these levels, the method includes determining a higher survival rate if all three of ER, AR, and VDR are found; determining an intermediate survival rate if only two of ER, AR, and VDR are found; and determining a lower survival rate if only one of ER, AR, and VDR are found. If none of ER, AR, and VDR is found, the method includes determining a mixed survival rate, with the survival rate being less favorable within the first five years after diagnosis, and leveling off thereafter to be very favorable by twenty years after diagnosis. While these survival rates are not absolute, the method of predicting a prognosis is largely based on the Kaplan-Meier survival curves shown in Figure 19C and 19D, as determined by the present studies explained in Example 10 below.

Since the classification system and survival rates for breast cancer are based on levels of ER, AR, and VDR present in a breast cancer tumor or cancerous tissue, treatment regimens aimed at affecting these proteins should be helpful in treating and/or managing breast cancer. As mentioned previously, "treating" involves arresting or inhibiting, or attempting to arrest or inhibit, the development or progression of a disease and/or causing, or attempting to cause, the reduction, suppression, regression, or remission of a disease and/or a symptom thereof. Accordingly, the present invention is further directed to a method of determining a treatment regimen for a breast cancer patient which includes measuring the levels of ER, AR, and VDR, and including at least one affecting molecule for each of ER, AR, or VDR present in the tumor or cancerous tissue. Accordingly, the treatment regimens and methods of treatment of the present invention are contemplated to be personalized to each patient, depending on which category and subclass their particular tumor or cancerous tissue is classified in based on the present classification system.

For instance, the method involves including in the treatment regimen at least one ER antagonist when ER is present in the tumor or tissue. Estrogen is a growth suppressor in normal breast cells. In contrast, it promotes growth in breast cancers. Thus, the role of estrogen is reversed in normal versus cancerous cells, so it is important to block the action of estrogen in cancer. As defined previously, an "antagonist" blocks or inhibits the effect of binding action on a receptor. Estrogen receptor (ER) and the estrogen signaling plays a role in breast cancer, leading to the proliferation of cells. By inhibiting the action of ER, such as with an antagonist, the downstream proliferation or growth of breast cells is also inhibited, thereby slowing the development and/or progression of breast cancer. For those tumors or cancerous cells that express ER, treatment with an ER antagonist such as but not limited to ICI 182,780 (Faslodex) inhibits proliferation of breast cancer growth. See Example 12 and Figure 24 for further details.

The method of determining a treatment regimen further involves including in the treatment regimen at least one AR ligand when the tumor or cancerous cells express AR. Contrary to estrogen, which reverses its role in cancer as discussed above, androgens affect cell growth in both normal and cancerous cells differently in patients depending on whether they also express HER2. Therefore, promoting the action of androgens is beneficial to inhibit cancer growth in some cases, and inhibiting androgens is beneficial in other cases. Specifically, in HER2+ and HER2- patients AR plays opposing roles. In HER2+ patients AR needs to be inhibited, and so an AR antagonist is used. In HER- patients, AR should be promoted so an AR agonist is used. As defined above, an "agonist" binds a receptor so as to trigger a biological response. Androgen receptor (AR) and the hormone androgen also play a role in breast cancer through signaling pathways that ultimately lead to inhibiting cellular growth. An AR agonist, such as but not limited to R1881 (Methyltrienolone) applied to breast cancer cells containing AR therefore inhibits further breast cancer cell proliferation and growth. See Example 12 and Figure 24 for further details. However, in HER2+ patients AR plays an opposite role and needs to be inhibited. So, in HER2+ patients AR antagonist is used to inhibit cellular growth. Therefore, as used herein, "ligand" means any molecule that will bind with the target receptor protein, such as AR, and encompasses both agonists and antagonists, depending on the breast cancer subtype.

The method of determining a treatment regimen also involves including in the treatment regimen at least one VDR agonist when the tumor or cancerous cells express VDR. As with androgens, Vitamin D also inhibits cellular proliferation in normal and cancerous cells. VDR agonists, such as but not limited to Calcitrol, therefore also inhibit cellular proliferation in breast cancer. See Example 12 and Figure 24 for further details.

Moreover, when more than one of ER, AR, and VDR are detected in the tumor or cancerous tissue, the method of determining a treatment regimen includes including a multi-hormone approach to the treatment regimen. As an illustrative example, if the tumor is ER+/VDR+, then both an ER antagonist and VDR agonist may be included in the treatment regimen. Such multi-hormone treatment has been shown to have an at least an additive effect, and in some cases a synergistic effect, producing even more robust inhibition of breast cancer cell growth when administered in combination than either hormone treatment product would achieve alone. See Example 12 and Figure 24 for further details. Any appropriate combination of multi-hormone treatment for the corresponding expression profile of ER, AR, and VDR is contemplated within the various embodiments of the instant invention. In at least one other embodiment, the treatment products do not act on ER, AR, or VDR specifically, but rather act on "partner proteins" to affect signaling in the same manner as if ER, AR, and/or VDR were directly acted on.

The present invention is novel in its use of multi-hormone therapy treatment for breast cancer, and offers many benefits over known cancer treatments. For instance, the present method of treatment is not chemotherapy, and therefore is safer and results in fewer side effects. Moreover, lower doses of each of the relevant hormone treatment products may be used to achieve an effective result, thereby being more tolerable and causing still fewer side effects. If multiple treatment products are called for according to the classification of the breast cancer, the treatment products may be administered to the patient concurrently for quicker and/or additive or synergistic effect. In other embodiments, the multiple treatment products may be administered to the patient in sequence, potentially rotating through one at a time, in order to further reduce any negative effects.

Moreover, the present treatment method provides benefits over other treatment protocols. For example, androgens have been known to induce early menopause in some women, which can be quite uncomfortable physically and emotionally. Vitamin D is important in calcium signaling, so affecting vitamin D receptor can have negative effects on calcium utilization in the body, and may lead to decreased bone porosity and osteoporosis. However, by providing treatment products that block ER signaling and promote AR and VDR signaling, particularly when administered in combination, the present invention provides better outcomes than when either are used alone, and may decouple the side effects from oncogenic effects.

As explained previously, currently breast cancers are classified based on the presence or absence of ER+, HER2+, or triple negative for ER, PR and HER2. Moreover, nearly 95% of HER2+ breast cancer tumors express at least one hormone receptor. Accordingly, in at least one embodiment the present method of determining a treatment regimen also involves including at least one HER2 inhibitor to the treatment regimen. One example is Lapatnib (Lap), although any HER2 inhibitor may be used. This is in addition to inclusion of at least one of the ER antagonist, AR agonist, and/or VDR agonist in the treatment regimen when these respective receptors are present or detected in the tumor or cancerous cells. In such embodiments, the HER2 inhibitor acts additively with the ER antagonist, AR agonist, and/or VDR agonist to inhibit breast cancer cell proliferation in the tumor or tissue.

Depending on the severity of the cancer and/or its stage, progression, and degree of aggressiveness, it may be helpful to include other more traditional cancer treatments in the treatment regimen in combination with the above-noted hormone treatment(s). Accordingly, in some embodiments, the method of determining a treatment regimen further involves including chemotherapy in the treatment regimen. As used herein and as is commonly understood in the art, "chemotherapy" means any drug, pharmaceutical, molecule, compound, or agent that is used to treat cancer, and may be specific to the type of cancer, such as breast cancer. The dose of chemotherapy utilized herein, the number of doses, and the frequency of doses are determined by a physician or other health care professional, as chemotherapy drugs are inherently toxic. Many factors must be considered in determining whether, how much, and how frequently to include and/or administer chemotherapy drugs with this invention, such as but not limited to: the particular disease and/or cancer being treated and its severity and/or stage of development/progression; the bioavailability, activity, and toxicity levels (e.g., LD₅₀) of the particular chemotherapy drugs used; the route or method of administration and introduction site on the subject; the rate of clearance of the specific chemotherapy drug and other pharmacokinetic properties; the duration of treatment; inoculation regimen and frequency; drugs used in combination or coincident with the specific chemotherapy agents, such as ER antagonists, AR agonists, VDR agonists, and possible contraindications between and side effects from the same; the age, body weight, sex, diet, physiology and general health of the subject being treated, including essential bodily organs such as the liver and kidneys used in clearing the chemotherapy agents; and like factors well known to one of skill in the relevant scientific art.

In some embodiments, the method of determining a treatment regimen also involves including radiation of the tumor or cancerous cells in the treatment regimen. As used herein, "radiation" refers to energy applied to a tumor or cancer cells to kill the cancer cells. It may include X-rays or other forms of radiation. In some embodiments it is applied or directed to the patient from an external source. In other embodiments, the radiation involves brachytherapy in which a portion of radioactive material is inserted and/or implanted within a patient and thereby emits radiation on the cancerous cells from within the patient's body. The wavelength, intensity, duration, and frequency of the radiation utilized in the present invention may be any as is used for the treatment of cancer, and is specific to the patient and depends on a number of factors including but not limited to the age, body weight, sex, diet, physiology and general health of the subject being treated; other treatments provided in concert with the radiation therapy; possible side effects and the intensity thereof; and like factors well known to one of skill in the relevant scientific art.

As should be apparent, the present invention is also directed to a method of treating breast cancer, and which is based on the instant classification system. Specifically, the method of treating breast cancer includes measuring the level of ER, AR, and VDR in the breast cancer tumor or cells of a patient. As before, these levels may reflect zero expression, or varying levels of expression of the particular hormone receptor. In at least one embodiment, the method also includes classifying the breast cancer of the patient based on the expression levels of ER, AR, and VDR detected in the breast cancer tumor or cancerous cells. Such classifying is in accordance with the classification system of the present invention, and which is based on the phenotypes of normal breast cells.

The method further includes providing a therapeutically effective amount of at least one ER antagonist to the patient when ER is detected in the tumor or cancerous cells. As used herein, "providing" is equivalent to, and can be used interchangeably with "administering." The meaning of "therapeutically effective amount" as used herein is as defined above. The method further includes providing a therapeutically effective amount of at least one AR agonist to the patient when AR is detected in the tumor or cancerous cells, and providing a therapeutically effective amount of at least one VDR agonist to the patient when VDR is detected in the tumor or cancerous cells. When the tumor or cancerous cells express more than one of ER, AR, and VDR, the method includes providing or administering a therapeutically effective amount of at least one of ER antagonist, AR agonist, and VDR agonist simultaneously or in combination, for an additive effect of inhibition of breast cancer cell proliferation, as discussed previously.

In the instant method of treating breast cancer, any of the ER antagonist, AR agonist, VDR agonist, and HER2 inhibitor may be administered by any appropriate method, as identified in the definitions section above. For instance, they may be administered orally or intravenously, as illustrative examples. Moreover, they may be administered with a pharmaceutically acceptable carrier, as previously defined, which may be in liquid, solid, or vapor form.

In certain embodiments, the method of treating breast cancer also includes administering at least one dose of chemotherapy to the patient, which is discussed in greater detail above. In some embodiments, the method of treating breast cancer includes administering at least one dose of radiation to the patient, also discussed in greater detail above.

The present invention is further directed to kits useful for detecting ER, AR, and VDR in samples, and may optionally also test for HER2 as well. Such kits may be used on solid tissue samples, biopsies, thin sections or sheets of tissue such as formalin-fixed paraffin-embedded (FFPE) tissue, tissue microarrays, cells in culture or in a matrix, and other biological samples, although these are not intended to be part of the kit. The kits may be used on biological samples from living patients or cadavers.

One embodiment of the kit of the present invention is antibody-based for immunostaining and detection. Such a kit includes predetermined amounts of primary antibody capable of binding at least one binding site of ER, AR, and VDR, respectively, for detection thereof. Accordingly, the kit contains a primary antibody for detecting each of these hormone receptors. Preferably, the kit includes three separate primary antibodies, one for each hormone receptor. In some embodiments, the kit also contains a predetermined amount of primary antibody capable of binding at least one binding site of HER2 for detection thereof. The particular amounts of primary antibody included in the kit may depend on the contemplated number of tests for which the kit was intended and/or the size of samples to be used on.

As used herein, "primary antibody" means an antibody raised against a particular target molecule in order to recognize that target molecule, and which will bind that target molecule upon encountering the same. They bind their particular target with high affinity and specificity, and may be specific to a particular binding site, or portion of the molecule at which the primary antibody binds. The binding site may be a certain amino acid sequence (in the case of a protein), a certain three-dimensional structure of a protein (such as an α-helix, a β-pleated sheet, a loop, a β-bulge), or particular functional groups of a chemical molecule. The primary antibody may be raised in any appropriate animal, including but not limited to mouse, rabbit, goat, donkey, and sheep. The primary antibody may be monoclonal, made from clones of the same antibody-producing immune cell, or polyclonal, made from a mixed population of immune cells.

In at least one embodiment, the present staining kit also includes a predetermined amount of a secondary antibody capable of binding at least one primary antibody for ER, AR, and/or VDR. As used herein, "secondary antibody" refers to an antibody that binds to a primary antibody or fragment thereof, and possesses a reporter or other marker for visualizing and/or identifying its location, and thereby the location of the primary antibody and target molecule of interest. Examples of the marker or reporter include a fluorophore that fluoresces at a certain wavelength, and which may be distinct from other fluorophores for simultaneous detection of multiple antibodies. The primary antibody acts as the antigen or target molecule for secondary antibody binding. The secondary antibody binds with high affinity and specificity to its target, which may be a whole Ig molecule, or fragments of the primary antibody, such as portions of the heavy or light chain, Fc or Fab regions. The secondary antibody may be raised in any appropriate animal, including but not limited to mouse, rabbit, goat, donkey, and sheep, but is raised in a different animal from that producing the target primary antibody. The secondary antibody may be monoclonal or polyclonal. The secondary antibody may be visualized by any appropriate method and using any appropriate device which will detect the secondary antibody, such as but not limited to fluorometers or visually.

Another embodiment of the kit of the present invention uses probes to detect ER, AR, VDR, and/or HER2 proteins. The kit therefore includes at least a predetermined amount of a first probe capable of binding to any site on ER protein, a second probe capable of binding to any site on AR protein, and a third probe capable of binding to any site on VDR protein. The probes may bind their target protein extracellularly or intracellularly, and may bind with any level of specificity and affinity, though higher levels of specificity and affinity may be preferable in some embodiments. In some embodiments, the kit may also include a fourth probe capable of binding to any site on HER2 protein for detection.

The kit may further include a detection probe(s) to permit detection of the first, second, third, and possibly fourth probes for the proteins. In other embodiments, the first, second, third, and fourth probes may be engineered such that conformational changes on binding with the respective target protein permit detection, such as through fluorescence of the probe upon binding.

Similarly, in another embodiment the kit uses probes as described above, but which bind to ER, AR, VDR, or HER2 mRNA rather than protein. In still other embodiments, the kit is a bioassay kit used to test for the biological activity of ER, AR, VDR, and in some cases HER2. The bioassay kits contain probes for detecting specific ER, AR, VDR and/or HER2 biological activity, such as downstream effects of signaling pathways in which each receptor is involved, which may for instance involve detecting the presence of a metabolite, chemical, or cellular action, as but a few examples. In still other embodiments, the kits include probes capable of binding particular "partner proteins" of ER, AR, VDR, and/or HER2, rather than the receptors themselves.

Accordingly, the kit of the present invention may be used for immunostaining. In certain embodiments, the kit may also include a visualizing device to detect the presence of the secondary antibody. Examples include a hand-held or portable fluorometer. In at least one embodiment, the kit also includes instructions for using the contents of the same.

### EXAMPLES

The methods and compositions herein described and the related kits are further illustrated in the following examples, which are provided by way of illustration and are not intended to be limiting. It will be appreciated that variations in proportions and alternatives in elements of the components shown will be apparent to those skilled in the art and are within the scope of embodiments of the present invention. Theoretical aspects are presented with the understanding that Applicants do not seek to be bound by the theory presented. All parts or amounts, unless otherwise specified, are by weight.

### Materials and Methods

### Tissue samples

Paraffin blocks from surgical resection specimens of normal breast tissue and of breast tumors were obtained from the archives of Brigham and Women's Hospital (BWH) in accordance with the regulations for excess tissue use stipulated by the BWH institutional review board. The study was conducted according to the principles outlined in the Declaration of Helsinki. A tissue microarray (TMA) of triple negative tumors (HTMA114) was previously described (54). TMAs BRC1501 and BRC1502 were purchased from Pantomics (Richmond, Ca). HER2 positive tumors were defined by IHC (expression >6 on scoring scale described below). A TMA of normal breast tissue and TMAs of samples from the Nurses' Health Study were previously described (36).

### Immunohistochemistry and immunofluorescence of tissues

Paraffin sections were stained with the antibodies and conditions indicated in Figure 11. Deparaffinized sections were blocked with 3% H₂O₂, antigen retrieval was performed using a pressure cooker with Dako citrate buffer (pH 6.0) at 120°C +/- 2°C, 15 +/- 5 PSI, slides were blocked with 3% serum and incubated with primary antibody (indicated dilutions in Figure 11) at room temperature for 40 minutes. Primary antibody application was followed by 30 minute incubation with Dako Labeled Polymer-HRP as a secondary antibody, and visualized with 3,3'-diaminobenzidine (DAB) as a chromogen (Dako Envision+ System). Mayer-hematoxylin was used as a counterstain. Immunostained sections were reviewed by light microscopy and scored visually with a value assigned to each individual core. Immunofluorescence was performed using similar conditions but with fluorescence labeled secondary antibodies conjugated with fluorescein isothiocyanate (FITC), Texas Red, Cy3, Cy5, or AlexaFluor dyes, and reviewed by standard fluorescence microscope.

In Figure 16, immunostained sections were scored independently by two pathologists using light microscopy and a 0 to 25 scale. The percent of tumor cells staining was quantified as (0) = 0%, (1+) = 1-20%, (2+) = 21-40%, (3+) = 41-60%, (4+) = 61-80%, and (5+) = 81-100%. The intensity of staining was quantified 0 to 5. The total score was calculated by multiplying the percent score with the intensity score. Therefore, there is a difference between expression and intensity. Expression is a quantal or bimodal determination of on or off, based on whether at least 1% of cells shows signs of expression. Intensity refers to how brightly the signal exhibits when present. The total score is based on both determinations.

Evaluation may be qualitative or quantitative in nature. For example, in the above embodiment, expression is considered when at least 1% of cells in the tissue show the protein of interest. In another embodiment, expression may be determined by at least 5% of cells exhibiting positive staining or other similarly appropriate detection technique. In another embodiment, the expression threshold is at least 10% of cells. In still another embodiment, expression is determined as at least 50% of cells showing positive detection. These above detection ranges apply equally to ER, AR, VDR, and HER2 detection.

For the Nurses' Health Study (NHS), in Figure 19 there were four cores for each of the 1,731 patients (6,924 cores in total). Tissue microarrays (TMAs) containing the cores were stained with ER, PR, HER2, VDR, AR, [K8/18/Cld-4], K5/6, and [SMA/p63/CD10] antibodies and scored semi-quantitatively. Given the enormous number of cores to be scored (1,731 x 4 x 7 = 55,392) and the bimodal expression pattern observed in pilot studies (Figure 16) we proceeded with a binomial scoring system with a 1% cut off point in this study. Scoring was 0 for no staining (<1%), 1 for positive staining. The pathologists were blinded to the scores given by the other pathologist and to survival outcomes. Scoring averages were determined per case using values assigned to all interpretable cores from the two pathology readings. If diagnostic tissue was absent or if the staining was not interpretable for all three cores, the case status was recorded as missing. ER, PR and HER2 status of each case was as previously described (58).

The NHS is a prospective cohort study initiated in 1976: 121,700 female US-registered nurses between the ages of 30 and 55 years completed a questionnaire covering factors relevant to women's health with biennial follow-up questionnaires used to update exposure information and ascertain nonfatal incident diseases. Information about the cohort, selection criteria for outcome analysis, covariates evaluated, and the statistical analysis methods are as previously described in the (36).

### Analysis of multiplex immunofluorescence

It has been very difficult to immunostain FFPE tissue sections with more than 3 different antibodies simultaneously for several reasons that involves limitations due to cross channel fluorescence of conjugates, incompatibility of primary antibody hybridization conditions and species limitation of secondary antibodies (59-61). First, while the excitation and emission wavelengths for fluorescence conjugates such as fluorescein isothiocyanate (FITC), rhodamine, Texas Red, Cy3, Cy5, and AlexaFluor dyes are theoretically non-overlapping, bleed through between channels is not uncommon. Thus simultaneous multiplexing requires use fluorescence conjugates with very different excitation and emission wavelengths, which limits the number of fluorescent probes that can be simultaneously used to 3 or 4, for all practical purposes. Second, primary hybridization of tissues with single primary antibodies versus with 3-4 different antibodies do not always produce the same results. This is because in some cases the antigen retrieval conditions are different for each antibody or optimum hybridization conditions may require different temperature or time. Thus one may have to use conditions that would work for all the antibodies simultaneously, which is many times is suboptimal for some of the antibodies, if not all (62). Third, most secondary antibody selectivity is species based, which also limits the number of secondary antibodies that can be multiplexed simultaneously, since most well characterized primary antibodies are produced in mouse, rabbit, rat or chicken. Thus, for all intents and purposes these put an upper limit to simultaneous multiplexing.

To solve these problems a sequential multiplex immunofluorescence method which was developed at GE Global Research Center (Niskayuna, NY) was used herein where two antibodies are hybridized to the tissue section at a time and images are collected with two laser exciting the tissue in two channel. Thus, this approach allows using the optimum antigen retrieval and hybridization conditions for each antibody and there is ostensibly no cross channel bleed-through of fluorescence (63). Briefly, IF-based sequential multiplexing method requires direct conjugation of primary antibodies with either Cy3 or Cy5 dye. Tissue sections were sequentially stained with two primary antibodies labeled with Cy3 and Cy5 for each round of staining, images were then acquired and then the fluorescent labels were inactivated. Another round of staining was then performed with another two Cy3 and Cy5 labeled antibodies. This process was repeated until all antibodies of interest were stained and images acquired. For this study, total of 12 antibodies were multiplexed on the same tissue section.

Image analysis was performed with MetaMorph 7.7 (Molecular Devices, Downingtown, PA). The results of the analysis of luminal epithelial cells are reported. The DAPI image was used to generate a nuclear mask image. A DAPI intensity threshold was set to identify all cell nuclei, and cut/join tools were used to outline each nucleus. Next, keratin negative areas were masked out to eliminate signals from stromal cells and the SMA image was used to mask myoepithelial cells. Integrated Morphometry Analysis / Measure Objects (IMA/MO) was used with minimum object area of 70 pixels to eliminate objects too small to be complete cell nuclei. The resulting Measured Objects image was binarized to generate a nuclear mask. The nuclear mask image was binarized, then dilated (neighborhood 2, repeat count 5, dilate without closing) to generate a binary cell mask image. The binary cell mask image was then applied to all eleven image planes using Arithmetic: source channels image stack and binary cell mask. The resulting image had the starting intensity values for every pixel the mask image was 'on', zero value for every pixel the mask image was 'off'. To ensure that every object appeared in every channel, the MetaMorph binary cell mask image was converted to a conventional 16-bit image with values 1 (where binary was 'on') and 0 (where binary was 'off'). This image was then added to each of the image channels, which resulted in equal minimum possible intensity value for each channel-object in each plane, and all image planes had identical objects. Next the data was exported to Microsoft Excel with threshold to outputs for object area and intensity (1 to 65,535). To facilitate analysis of double (HR2) and triple (HR3) hormone receptor positive cells, MetaMorph was used to generate images featuring HR2 and HR3 cells. Starting with the eleven plane image stacks of separated cell objects, the stack was duplicated, used Keep Planes command to keep the image plane channels of interest - ER, AR, VDR (nuclear) - and used Stack Arithmetic Average, to generate an image, "HR2" or "HR3", with bright nuclear signal if any of the ER, AR or VDR (nuclear) signals were high. For display purposes, Color Combine was also performed on some image triplets, ex. Red=ER, Green=AR, Blue=DAPI.

### Nurses' Health Study: design and population

The NHS is a prospective cohort study initiated in 1976: 121,700 female US-registered nurses between the ages of 30 and 55 years completed a questionnaire covering factors relevant to women's health with biennial follow-up questionnaires used to update exposure information and ascertain nonfatal incident diseases. Information about the cohort, selection criteria for outcome analysis, covariates evaluated, and the statistical analysis methods are as previously described (36).

### Analysis of mRNA expression profiles

Kaplan-Meier analysis of relapse free survival for all invasive breast cancers from the 855 patient breast tumor dataset (37) (https://genome.unc.edu/) was performed by first independently rank ordering all 855 tumors according to gene expression values for ER, AR, and VDR. Next, tumors were classified as 'Positive/High' or 'Negative/Low' for each gene, independent of each other using a 50th percentile cut off. The groups were then assembled based on positive or negative call for each gene; all negative=HR0, one positive=HR1+, two positive=HR2+, all three positive=HR3+.

### Characterization of breast cancer cell lines

All cell lines were cultured according to ATCC's guidelines. BT20, HCC1187, MDAMB468 and SUM159 cells were trypsinized with 0.05% trypsin/EDTA and plated at appropriate densities (BT20: 6,000, HCC1187: 10,000, MDAMB468: 4,000 and SUM159: 1,000 cells/well) in 96 well plates in DMEM+10%FBS. The next day Calcitriol and Taxol were added at concentrations of 25nM and 1.5nM respectively (Day0). For ZR75B, 4000 cells/well were plated in 96 well plates in DMEM+10%FBS. 50nM Calcitriol and 5nM ICI180,782 were added the next day with fresh medium (Day0). Media was refreshed every two days and cell proliferation assay was performed on day 6 for above cell lines. The T47D cells were trypsinized with phenol red free Triple Express (Invitrogen) and washed once with PBS and 10,000 cells/well were plated in phenol red free DMEM+ 5% charcoal stripped FBS (CSFBS) in 96 well plates and cultured for 3 days. On the fourth day, 10nM 17 beta estradiol (E2) was added into all the wells except non-drug treatment group, in addition to 10nM ICI180,782, 10nM R1881 and 50nM Calcitriol in different groups with fresh phenol red free DMEM+5% CSFBS (Day0). Cells were cultured in the same medium for 6 days before counting. All the proliferation assays were carried out using cell titer blue reagent (Promega) and fluorescence was measured using Bio-Tek spectrophotometer at 530/25nm (excitation) and 590/35nm (emission). Vehicle only group was used as control in all the experiments except for T47D, where E2 was used as control.

### Statistical Analysis

Event outcomes were compared using Kaplan-Meier analysis and P values were determined with the logrank test. Tumor expression of myoepithelial and luminal genes cells (25-27) were explored in fRMA normalized (McCall & Irizarry 2010, Biostatistics) gene expression data (GSE3744, , GSE4922, GSE6532, GSE7390) using hierarchical cluster analysis (Pearson correlation coefficient, average linkage) using the Bioconductor package made4 (Culhane et al. 2005). Global test (Goeman et al. 2004) was used to assess the association between gene expression and luminal or myoepithelial classification. Tumors were classified into basal or non-basal-like as previously described (Culhane & Quackenbush 2009), except where were basal-like subtype classification was provided by inventors (Richardson et al. 2006). Globaltest, which is available from Bioconductor package globaltest (Goeman, J. J., van. (2004)), was used to determine association between individual genes and the basal/non-basal division.

### Gene Expression Analysis

In order to develop the present classification system and methods of using the same, experiments were conducted on normal breast cells to use as a reference for classifying cancerous tumors or tissues. Specifically, systematic analysis of a large set of breast epithelial markers was conducted. This information was used to classify human breast tumors based on normal cell types into four major subtypes. This novel ontological classification scheme is associated with significant patient survival differences and provides actionable insights for treating breast tumors, as explained in greater detail in the Examples and Figures.

### Example 1: Analysis of Cluster of Differentiation (CD) Markers in Normal Human Breast

As noted previously, normal human breast is composed of milk producing lobules and interlobular ducts that transport the milk to the nipple (**Figure 1A**). The vast majority of human breast tumors are classified on morphological grounds either as **'ductal carcinomas"** or as **'lobular carcinomas',** resulting in a common misconception that ductal and lobular breast cancers initiate in the normal ducts and lobules, respectively. However, despite their names, almost all of the early progression steps for both tumor types almost exclusively involve the breast lobules. Thus, the normal cells in the lobules were examined using immnunohistochemical (IHC) staining that preserves tissue architecture and allows discrimination of ducts, lobules and different layers of the epithelium. However, most proteins are expressed in a gradient pattern *in vivo* which limits their utility to define cell subtypes using semi-quantitative methods such as IHC. Therefore, markers having bi-modal expression patterns (i.e. one subpopulation clearly negative and another strongly positive) were identified based on immunohistochemical staining of normal breast tissue (**Figure 1**).

### Example 2: Analysis of Intermediate Filaments in Normal Human Breast

In an attempt to identify molecules with bimodal expression patterns in normal human breast, the expression of intermediate filaments was examined since these molecules are differentially expressed in distinct cell types and their expression is both tissue and cell-type specific. Furthermore, it has been well recognized that the cell-type specific expression of intermediate filaments is preserved in tumors (10). For this reason, they have been successfully utilized by embryologists to study tissue differentiation and by pathologists to determine the tissue origin of tumors (11). A subset useful in identifying subpopulations of human breast cells was discovered which expressed in a bi-modal pattern. These included K5, 7, 8, 14, 17, 18, and 19 (**Figure 1J**).

Next, normal breast tissues from 36 breast reduction mammoplasty procedures was examined, including 12 full FFPE sections and a tissue microarray with samples from 24 patients. These cases were immunostained with K5, 7, 8, 14, 17, 18, 19, CD10, p63 and smooth muscle actin (SMA).

Normal breast lobules and ducts are lined by a bi-layer epithelium that is thought to consist of two cell types, an inner milk producing luminal cell-type and an outer supportive myoepithelial cell-type. As previously shown, K7, K18 and Claudin-4 (Cld-4) are expressed in all luminal layer cells and not expressed in the myoepithelial layer (**Figure 2A**) (12). Thus, these three antibodies constitute a pan-luminal marker panel that identifies all luminal cells. In contrast, CD10, SMA and p63 are expressed in all myoepithelial layer cells, but not in luminal layer cells; thus, they constitute a pan-myoepithelial marker panel. Interestingly, while a majority of luminal cells stained with K19, in rare lobules K19 was negative (**Figure 2B**). Thus, while exclusively luminal in its expression pattern, K19 is not a pan-luminal marker.

In human skin and mouse mammary tissue K5/14/K17 are exclusively expressed in the basal layers of skin the myoepithelial layer of breast (**Figure 3A**,**B**). Based on these observations these keratins are usually referred to as 'basal keratins' (13). However, in normal human breast tissue K5, K14 and K17 were not exclusive to a single layer; their expression switched between luminal and basal layers depending on location. In the larger caliber interlobular ducts in human breast K5/14/17 were expressed in the myoepithelial (basal) layer as expected (**Figure 1B**, **3C-E**). However, in the lobules, the site where early progression steps for breast cancer and precursor lesions develop, K5, K14 and K17 were more prominently expressed in the luminal layer (**Figure 2C-E**, **1J**, **3F-H****)** (7). The luminal nature of these [K5+, K14+ or K17+] positive cells was confirmed with double immunostains. The [K5+, K14+ or K17+] cells in the lobules were located above the vimentin+, SMA+, CD10+ and p63+ myoepithelial cell layer and directly facing the luminal space. These cells were also negative for the myoepithelial specific markers CD10, SMA and p63, confirming their luminal phenotype (**Figure 3I-K**).

Luminal [K5+, K14+ or K17+] cells were identified in all patients examined (n=36). Thus, this was a robust and highly reproducible subpopulation of luminal cells. Of note, while in some lobules only a small percent of luminal cells were [K5+, K14+ or K17+], in other lobules, nearly 100% of luminal cells were [K5+, K14+ or K17+] even in a single section of tissue from the same patient (**Figure 3L-O**).

When two different cell lineages are defined by mutually exclusive expression of markers, co-expression of these markers in the same cell has been used as evidence of 'stemness'. Previously, co-expression of K5, K14 or K17 with K7 or K18 has been interpreted as evidence for bi-potential cells with a mixed luminal/myoepithelial phenotype. The evidence here, however, demonstrates that some lobules are entirely composed of such double-positive cells and such lobules are present in nearly every tissue section examined (**Figure 2F****,** **Figure 3P-T**). Importantly, these K5+ and K14+ cells also expressed Muc1, a marker of luminal differentiation (**Figure 3U**). Since it would be extremely unusual to find an epithelial tissue that is entirely composed of progenitor/stem cells, these results indicate that the luminal layer cells that co-express K5/14/17 with K18/19 are more consistent with a differentiated luminal cell variety.

These data show that keratins 5/14/17 are not exclusively 'basal keratins' in human breast, unlike their uniform basal expression in human skin and mouse breast. In the lobules of normal human breast K5/14/17 are predominantly expressed in the luminal layer. In contrast, in the interlobular ducts, K5/14/17 are predominantly expressed in the myoepithelial (basal) layer (7, 14). Notably, K5/14/17 expression was bi-modal within the luminal layer, i.e., there were two distinct luminal layer cell populations with one subset that is K5, K14 and K17 negative, and another subset that is K5+, K14+ or K17+. In all of these studies, each marker was examined with several antibodies that were from different species, manufacturers and clones to rule out antibody isoform specific variables. In all cases the results were similar between different antibodies (see **Figure 11** for a list of 37 primary antibodies used in these studies).

### Example 3: Analysis of Hormone Receptors in Normal Human Breast

Having identified two subtypes of luminal layer cells based on K5/14/17 expression, the expression of hormone receptors in these cells was characterized (15). Hormone receptors represent an appealing set of markers because they are intimately involved in regulation of tissue differentiation and some hormone receptors have a bi-modal expression pattern.

In an initial survey of the previously published studies and preliminary immunostains, three receptors including the estrogen receptor (ER), androgen receptor (AR) and vitamin D receptor (VDR) stood out with a distinct bi-modal expression pattern in luminal layer of the lobules (strong expression in some cells while other cells have no expression). Many of the other hormone receptors did not appear to have a bi-modal expression pattern (TRH α/βPTH1R, OXTR, SSTR1-3,5, RAR α/β, and RXR α/β). Thus, these other receptors did not appear promising as potential bimodal markers to identify cellular subtypes in normal human breast. Because expression of the progesterone receptor tracks with expression of ER, PR was not included in the instant characterization of the already extensive panel of markers.

Double immunostains with K5/14/17 and ER were next conducted. This analysis demonstrated that ER and K5/14/17 were not co-expressed in the same cells (<0.5% overlap, **Figures 5-9**) (**Figure 2G****,** **Figure 1J****,** **Figure 5**), therefore defining three mutually exclusive luminal cellular states: **(a)** ER+, **(b)** K[5/14/17]+ and **(c)** ER-/K[5/14/17]- (**Figure 2H**). Staining of the same sections with a Ki-67 antibody which labels proliferating cells regardless of their location within the cell cycle revealed that the proliferating luminal cells very rarely expressed ER or K5/14/17 (<0.4% overlap, **Figure 6**) (**Figure 2I-J**). Almost all proliferating Ki-67+ cells were K18+ luminal cells (**Figure 2K**). These results allowed us to define four mutually exclusive luminal cell states in the lobules of normal breast: **(a)** ER+ cells, **(b)** K[5/14/17]+ cells, **(c)** ER-/ K[5/14/17]- cells and **(d)** Ki-67+ cells (**Figure 2L**). All of these cells were positive for the pan-luminal markers K7/18.

Double immunostains demonstrated that AR+ cells were also mutually exclusive with K/5/14/17+ and Ki-67+ cells (0.0% overlap, **Figure 5****,** **Figure 4A and B**), but they did partially overlap with ER+ cells (11-36%, **Figure 7**, **Figure 4C**). These results indicated three subsets of hormone receptor (HR) positive cells: ER+, AR+ and AR/ER+ (**Figure 4D**). AR expression was only seen in the luminal layer and not in the myoepithelial layer.

Double immunostains also demonstrated that VDR+ cells were exclusively in the luminal layer as well, with no overlap with CD10+ myoepithelial cells (**Figure 4E**). VDR+ luminal cells were also mutually exclusive with proliferating Ki-67+ cells (0.0% overlap, **Figure 6****,** **Figure 4F**), but they did partially overlap with K5/14+ cells (15-23%, **Figure 5****,** **Figure 4G****),** AR+ cells (16-35%, **Figure 7****,** **Figure 4H**), and ER+ cells (22-74%, **Figure 7****,** **Figure 4I**). Interestingly, the vast majority of the Ki-67+ proliferating cells were in the luminal layer, as evidenced by their being mutually exclusive with CD10+ myoepithelial cells (**Figure 4J**, **Figures 5-9****)**. A triple stain also demonstrated the presence of triple HR+ (ER/AR/VDR+) cells (**Figure 4K**,**L**, white triple positive). The proliferating luminal cells were mutually exclusive with hormone receptor expressing ER, AR or VDR cells **(****Figure 4M****)**.

These results indicated seven subsets of hormone receptor (HR) positive cells in the luminal layer of human breast lobules: ER+, AR+, VDR+, AR/ER+, ER/VDR+, AR/VDR+ and ER/AR/VDR+ that are depicted in a diagram in Figure 4N. Interestingly, only VDR+ cells substantially overlapped with K5/14/17+ luminal cells, and the proliferating (Ki-67+) luminal cells were negative for all of these markers (**Figure 4B**, **F**, **M**).

Cumulatively, 11 differentiation states were therefore defined in the luminal layer of human breast lobules (L1-11; **Figure 10****).** These include three hormone receptor negative states (**HR0; Ll-3 in** **Figure 10**) and eight hormone receptor positive (HR+) states (**L4-11 in** **Figure 10**), grouped as single hormone receptor positive (**HR1+:** ER, AR or VDR; L4-L7), double hormone receptor positive (**HR2+:** ER/AR, ER/VDR, or AR/VDR; L8-L10) or triple hormone receptor positive (**HR3+:** ER/AR/VDR; L11) (**Figure 10**). All luminal cells expressed K7/18 and Cld-4.

In the myoepithelial layer, all cells expressed SMA, CD10 and p63, with two subtypes that were either K5/14/17[-] or K5/14/17[+] (**Figure 10**, My1 and My2). Proliferating cells are very uncommon in the myoepithelial layer; CD10 and Ki-67 overlapped in only 0.5% of the cells (**Figure 4J****,** **Figure 10****,** **Figure 11D**).

### Example 4: Simultaneous Examination of Twelve Markers in Normal Human Breast with a Novel Multiplex Immunofluorescence Method

In the above experiments the same FFPE section was stained with up to three different antibodies simultaneously. A greater number of antibodies are difficult to multiplex using conventional methods for multiple reasons discussed above. Staining serial sections does not address these problems because each section is 5 microns thick, hence the same cell is not present in more than two serial sections at best.

To confirm simultaneous co-expression patterns predicted by the double and triple immunostains for all twelve different markers ER, AR, VDR, K5, K7, K8/18, Cld-4, SMA, CD10, Ki-67, NaKATPase and DAPI, their expression in the same cells was examined by multiplex immunostaining.

Specifically, a new technology has recently been developed that allows immunostaining of the same tissue section with more than 10 different antibodies serially (General Electric, NY, USA) (16). This technology was used to confirm the above results by staining tissue microarrays (TMAs) containing normal human breast lobules from twenty-four patients, according to the Materials and Methods section. In these experiments a pan-keratin stain was used to identify all epithelial cells. DAPI and NaKATPase were used to highlight nuclei and membranes, respectively. These experiments independently confirmed co-expression patterns of all markers in Figure 10 (**Figures 12-14**). Once again, proliferating luminal cells did not express hormone receptors (ER, AR and VDR), or Keratins 5/14. Hormone receptor positive cells were exclusively in the luminal layer and they partially overlapped with each other conforming to the HR1, HR2 and HR3 phenotypes we previously observed (**Figures 12-14**).

Among the 11 differentiation states of the present invention described for normal luminal cells in human breast, these experiments highlight four predominant differentiation patterns: a hormone receptor positive state (HR+), a proliferative state (Ki-67+), and two hormone receptor negative states; one K5+ and another K5- (**Figures 13-15**). Since these are by and large mutually exclusive states it was hypothesized that if one phenotype expanded others must shrink in a lobule. Consistent with this, Ki-67 and K5+ cells were rare in lobules enriched in HR+ cells (**Figure 15** **ii-iii**). When K5+ cells expanded, HR+ and Ki-67 cells decreased (**Figure 15** **iv-v**) and, in highly proliferative areas, there were very few HR+ or K5+ cells (**Figure 15** **vi**). Hence, the multiplex staining technology on single sections confirmed that these differentiation states are largely mutually exclusive, where a given cell can exist in only one state at one time (**Figure 15** **vii**) (17).

### Example 5: Analysis of HR+ and HR- Cell Types in ER+ Breast Tumors

Given the remarkable heterogeneity observed at the single cell level in normal breast epithelium, whether breast tumors maintain normal cell type/differentiation specific patterns was investigated.

In a preliminary set of 20 breast cancers, first the staining pattern of twelve protein markers in full FFPE sections was evaluated with IHC (**Figure 11**). These results were confirmed in tissue microarrays (TMAs) that contained a second set of 216 tumors (including 51 ER+, 46 HER2+ and 119 TNBC) (**Figure 16**). The staining of tumors in the TMA was quantified as (0) = 0%, (1+) = 1-20%, (2+) = 21-40%, (3+) = 41-60%, (4+) = 61-80%, and (5+) = 81-100%. The intensity of staining was quantified 0 (no staining) to 5 (maximum intensity). The total score was calculated by multiplying the percent score with the intensity score to derive a 0-25 expression scale (**Figure 17**).

In both tissue sets, all ER+ human breast cancers strongly expressed multiple pan-luminal markers (Cld-4, K7 and K18) and none were positive for pan-myoepithelial markers (CD10, SMA and p63) (**Figure 16A**). All ER+ breast cancers were also negative for K5/14 (**Figure 16A**). Interestingly, the majority of ER+ tumors were VDR+ (93%), and two thirds were AR+ (59%) (**Figure 16A**). This pattern was identical to the normal breast ER+ cells that can co-express AR or VDR, but are very rarely positive for K5/14/17 or myoepithelial markers CD10/SMA. These results indicate that all ER+ tumors have a luminal phenotype identical with HR+ normal breast cells in the luminal layer (normal differentiation states L4, L8, L9, and L11) (**Figure 16A and D**). Intriguingly, similar to normal tissues, most proliferating tumor cells (Ki-67+) were also negative for ER or AR (**Figure 18B-I**). While most Ki-67+ tumor cells were VDR negative (**Figure 18J-M**), focally some proliferating tumor cells were also VDR+ (**Figure 18N-Q**).

### Example 6: Analysis of HR+ and HR- Cell Types in HER2+ Breast Tumors

In HER2+ tumors, strong expression of multiple pan-luminal markers (Cld-4, K7 and K18) were observed, and none of the pan-myoepithelial markers (CD10, SMA, p63) were present **(****Figure 16B****).** These data indicated that nearly all HER2+ tumors (44/46) have a HR+ luminal phenotype (L4-L11) that is identical with normal breast cells (**Figure 16B**,**D**). A minority (2/46) were similar to HR negative cells (**Figure 16B**,**D**). One possible explanation of why there so few HR0 HER2+ tumors is that HER2 overexpression may be detrimental in HR0 cells, while it is beneficial to HR+ cells tumors.

### Example 7: Analysis of HR+ and HR- Cell Types in Triple Negative Breast Tumors

Triple negative breast carcinomas (TNBC) are defined as lacking expression of ER, PR and HER2. For the present invention, 119 TNBC were analyzed for the expression of HRs and keratin markers. This work revealed three major subgroups referred to as Luminal 1 (LM1), Luminal 2 (LM2) and Mixed (M) **(****Figure 16C****)** based on staining for K5/14 (18). Nearly 66% of the TNBC (n=78/119) had a pure luminal phenotype; the phenotype of these tumors was similar to that of normal luminal cell types that are positive for pan-luminal markers, negative for pan-myoepithelial markers and ER, but positive for AR, VDR or both (**Figure 16C**). Among these, 37 were identical to the luminal cell types that are K5/14 negative (L1-2, L5-7, or L10 cell types (LM1) and 41 identical to normal luminal cell type L3 or L7 that are K5/14 positive (LM2) (**Figure 16C****,D**).

All of the remaining TNBC (33%, n=41) also strongly expressed luminal markers (Cld-4, K7, AR, VDR, K18), but 38 of these tumors expressed antigens found in normal myoepithelial cells (CD10, SMA and p63) (**Figure 16C**). These tumors have a phenotype that is almost equal parts of luminal and true basal phenotypes (i.e. they express *bona fide* basal/myoepithelial markers), hence they are referred to as "mixed phenotype" (M).

These data show a surprisingly high fraction of human breast tumors (∼95%) are phenotypically identical to a single normal breast cell sub-type, indicating that these tumors either retain the differentiation phenotype of their cell-of-origin or they differentiate along normal cell differentiate lineages. The normal counterpart cell type could not be identified for a small minority (∼5%) of human tumors with mixed differentiation. It is possible that the normal counterpart of these tumors might be a cell type such as progenitor cells with mixed glandular and myoepithelial(19), or these tumors may exhibit an altered phenotype due to mutations that result in inappropriate expression of these markers.

### Example 8: Expression of normal basal vs. luminal specific mRNAs in TNBC

The cell-of-origin of TNBC has been of great interest (20). As mentioned earlier, keratins K5/14/17 are expressed in the basal layers of human skin and rodent mammary glands (**Figure 3A****,B**). Thus, these keratins have been commonly referred to as 'basal keratins' in the literature (14). Consequently, some of the TNBCs that express K5/14/17 have been called 'basal-like carcinoma' (BLC) (21). Based on this, some have suggested that these tumors are similar to myoepithelial (basal) cells of the normal breast. However, as demonstrated herein, K5/14/17 are predominantly expressed in the luminal layer of normal human breast lobules and K5/6+ TNBC basal-like cancers (BLC) express markers identical to those in these luminal cells (**Figure 10**, L3 and L7).

Because the TNBC/BLC category was based on mRNA expression in microarray analysis (22-24), an analysis of mRNA in normal human breast cells was conducted, by combining results from three different studies that profiled highly purified luminal vs. myoepithelial cells (25-27). Only a small subset of mRNAs was differentially expressed between luminal and myoepithelial cells consistently in all three studies. These provided a strong consensus signature that distinguished normal luminal vs. myoepithelial cells. The expression of these genes was then examined in basal-like and non-basal-like human breast tumors (28-31).

Interestingly, no significant correlation was observed between 'basal-like tumors' in these cohorts and the expression signature of normal basal/myoepithelial cells (**Figure 20A**). In fact, the list of genes in the three previously published 'luminal' or 'myoepithelial' signatures did not significantly overlap with genes that were more either strongly expressed in basal or non-basal tumors (Fisher's exact test P value = 0.22) (**Figure 20A**). Thus, most "basal-like" tumors did not demonstrate true basal phenotypes as defined by expression of mRNA and protein markers of normal myoepithelial cells, the true basal cells (**Figure 20B**). Intriguingly, recent studies in mouse models have also suggested that the cell-of-origin of basal-like carcinomas is in fact a luminal cell (14, 20, 32). In some cohorts, patients with basal-like tumors have a worse outcome than those with TNBC tumors (21). In the present analysis, there was no observable significant difference between K5/6+ vs. K5/6- TNBC patients (**Figure 20A**).

These data show that while basal-like tumors are a distinct and reproducible sub-group of TNBC based on mRNA profiling, the name "basal-like' is probably not an accurate description of their differentiation state or their cell-of-origin (7, 14, 33). The differentiation state of BLC is most similar to K5/14/17[+] normal luminal cells of the breast.

### Example 9: Distribution of HR0-3 Breast Tumor Phenotypes in the NHS Cohort

Based on the above results, it was hypothesized that human breast tumors can be classified according to normal differentiation states of human breast tissue. This hypothesis was tested using a breast cancer cohort from the Nurses' Health Study (NHS) with patient follow up extending beyond 25 years, and availability of breast cancer samples from a large number of patients (n=1731) (34-36). Tissue microarrays (TMA) including samples from 1,731 NHS patients were immunostained with ER, PR, HER2, VDR, AR, K8/18/Cld-4, K5/6, and SMA/p63/CD10 antibodies and scored semi-quantitatively. The pilot study had revealed that these markers are expressed in a bimodal pattern in the vast majority of tumors (**Figure 16**). Thus, given the enormous number of cores to be scored (>55,000), a binomial scoring system was implemented with a 1% expression cut off point when scoring the NHS TMA. In this study the breast tumors were divided into four categories based on normal tissue differentiation: triple-positive tumors (**HR3**) that co-express ER, AR and VDR, double-positive tumors (**HR2**) that are ER/AR[+], AR/VDR[+], or ER/VDR[+], single-positive tumors (**HR1**) that only express one of the hormone receptors, ER[+], VDR[+] or AR[+], and hormone-receptor negative tumors (**HRO**) that are negative for ER, AR and VDR.

Notably, the four HR categories are not identical to the current ER+, HER+ and TNBC classification. For example, based on standard classification about 75% of NHS study patients had ER+ tumors, (n=1, 356), 10% had HER2+ tumors (n=177) and 15% triple-negative tumors (n=253) (**Figure 19A****,B**). In contrast, these same tumors were classified as HR3 58.1% (n=1006), HR2 24.8% (n=429), HR1 10.7% (n=185), and HR0 6.4% (n=111) (**Figure 19A****,** **Figure 25**).

Examination of each standard breast cancer category revealed that each subtype is composed of multiple HR groups. For example, HR classification of ER+ tumors revealed that 75% were HR3+ (ER+/VDR+/AR+), and that the remainder of ER+ tumors consisted of HR2+ (23.4%) and HR1+ (1.5%) tumors (**Figure 21A****,B**). Nearly one third of HER2+ tumors (29%) expressed all three hormone receptors (HR3+), the remainder comprised of HR2+ (43.5%), HR1+ (22.0%) and HR0 (5.1 %) tumors (**Figure 21A****,B**). Thus, a HR based classification approach does not merely rename existing groups, but it organizes breast tumors in a new way. As a result, the distribution of HR0-3 patients is significantly different than the current categories. Among triple-negative breast cancers (n= 262), 36.8% did not express any hormone receptor (H0), 44.6% expressed at least one hormone receptor AR or VDR (HR1+), and 18.6% expressed both AR and VDR (HR2+) (**Figure 21A****,B**).

### Example 10: Analysis of Breast Cancer Outcomes Based on Normal Cell Lineage Phenotypes

There is a strong association between the total number of positive receptors in the HR0-3 categories and breast cancer survival or outcome. Specifically, Kaplan-Meier analyses of the NHS cohort showed that women with HR3+ tumors have the best survival, HR1+ tumors have the worst survival, and HR2+ tumors had an intermediate survival (**Figure 19C**) (p <0.0001).

In multivariate analysis these differences remained significant. When compared to HR3 tumors, the relative hazard ratio (RHR) for HR2 tumors was 2.9 (95% confidence interval [CI] 1.60-5.21), for HR1 tumors the RHR was 5.3 (95% CI 2.77-9.97) and for HR0 the RHR was 6.9 (95% CI 3.37-14.39).

Interestingly, the HR0 group had biphasic outcome curve that was similar to HR1 tumors with the worst outcome during the first five years, however, after 5 years the curve was flat consistent with an excellent outcome (**Figure 19C**). A key assumption of the Cox regression model is a constant hazard ratio over time. Thus, we added time-dependent co-variables in the multivariate model and re-evaluated the association stratified by time before and after a five year cut off. During the first five years HR3 tumors had the best outcome, HR2 tumors had a worse outcome compared to HR3 (RHR=1.69, 95% CI=1.14-2.50) and both HR1 and HR0 tumors had the worst outcome (HR1 RHR = 2.44, 95% CI = 1.55-3.84; HR0 RHR = 2.7, 95% CI = 1.56-4.70) (p <0.0001) (**Figure 21C****,** **Figure 26**). After five years there was no significant difference between HR3, 2 and 1 (p >0.5), except that HR0 group that had a better outcome hazard ratio 0.34 (p=0.02) (**Figure 21D**). Analyzing the HER2 groups separately did not change these results (**Figure 22E**). In a multivariate analysis these differences remained significant even after accounting for other factors in a multivariate analysis, such as age, stage, grade, HER2 status, treatment and radiation (**Figures 25-26**).

These survival results were confirmed at the mRNA expression level by examining a dataset of gene expression microarrays from 855 human breast tumors (37). The Kaplan-Meier analyses for relapse free survival showed that women with HR3+ tumors had the best outcome, HR1+ and HR0 tumors were the most aggressive, and HR2+ tumors were intermediate in between these groups. Unlike the IHC based HR categories that had very significant overall survival differences (p < 0.0001, **Figure 19C**), there was a more modest overall relapse free survival difference among mRNA based HR groups (p= 0.13, **Figure 19D**). However, the lung relapse free survival differences between mRNA based HR groups was significantly different (p= 0.0014, **Figure 20F**).

Taken together, these data support that the total number of hormone receptors correlate with the differentiation state of the tumor cells, and more differentiation correlates with less aggressive tumor behavior. Importantly, these results suggest that in the case of ER, AR and VDR, measurement of protein expression levels may be more relevant than mRNA levels for predicting tumor behavior.

### Example 11: Analysis of HR+ and HR- Cell Types in Breast Cancer Cell Lines

The preservation of HR0-3 phenotypes in breast cancer cell lines was also examined. Publicly available mRNA expression data from over 60 breast cancer cell lines (38) was analyzed, which revealed that most tumor cell lines fall into one of the normal cell-of-origin categories.

Specifically, the HR+ pure luminal breast cancer cell lines (ER/AR/VDR+, n=16) rarely expressed K5/14/CD10/SMA, as expected (**Figure 23G**). This was also true for the HER2+ tumor cells lines (n=13), that rarely expressed K5/14/CD10/SMA but were occasionally AR/VDR+ as expected. There were five cell lines that had a typical TNBC-LM1 phenotype (BT-20, HCC38, HCC-1187, SUM149), three cell lines with a TNBC-LM2 phenotype (BPLER, HCC-1143, HCC-1500), and 5 cells lines had a mixed phenotype (HMLER, HCC-70, HCC1937, HCC-3153, MDA-MB-468) (**Figure 23G**). These phenotypes were also confirmed at the protein level in breast cancer cell lines (**Figure 23H**), in order to select a subset of breast cancer cell lines that closely conform to *in vivo* HR phenotypes (**Figure 23I**). This set of breast cancer cell lines was examined for *in vitro* drug response studies described below.

Interestingly, a number of cell lines such as MDA-MB-231, SUM-159, MDA-MB-435 (n=9) that are very frequently used as models of human breast cancer had an expression profile that was not present either in normal breast cells or in human breast cancers. These particular cell lines were negative for most of the epithelial markers surveyed in the present studies. Since this profile is almost never seen *in vivo,* it seems that either these cells have lost their original phenotype or they were derived from very rare tumor types. Thus, among all the cell lines examined, these particular cell lines appear to have the lowest resemblance to most common forms of human breast cancers, cautioning against their frequent use as typical models of the human disease.

### Example 12: Response of Breast Cancer Cell Lines to HR Inhibition

The HR0-3 classification of breast cancers not only correlates with clinically significant outcome groups, but also provides insights about how the treatment of these patients might be personalized. For example, the present invention contemplates HR3 tumors might be treated with a triple-hormone therapy, combining ER antagonists with AR and VDR agonists. Some of these concepts were therefore tested in breast cancer cell lines.

There are very few effective treatments against TNBC currently because they are negative for ER and HER2. But, because 63% of TNBC express either AR or VDR, or both receptors, hormone treatment might be possible in a majority of TNBC, in combination with chemotherapy. The BT20, HCC1187, MDA-MB-468 and SUM159 only express VDR receptor corresponding to TNBC-HR1 phenotype. Combining the VDR agonist Calcitrol with Taxol additively inhibited proliferation of these HR1+ breast cancer cells more effectively than either drug alone **(****Figure 24J****).**

A similar combination treatment strategy can be also employed in ER+ tumor cells. For example, the HR2+ ZR75B cells co-express ER and VDR receptors, and combining a VDR agonist Calcitrol with low doses of ER-antagonist ICI 182,780 (Faslodex, ICI 0.5nM) additively inhibited proliferation of these breast cancer cells (**Figure 24K**). In another example, the combination AR-agonist R1881 (Methyltrienolone, 50nM) and VDR agonist Calcitrol (Cal, 50nM) additively inhibited proliferation of HR3+ breast cancer cell line T47D (**Figure 24L**).

In HER2+ breast cancer cells, the combination AR-antagonist Flutamide (Flu 45µM) and HER2 inhibitor Lapatnib (Lap 0.5µM) additively inhibited proliferation of the HR2+/HER2+ breast cancer cell line MDA-MB-453 (**Figure 24M**). Similarly, the combination of the ER-antagonist ICI 182,780 (Faslodex, ICI 0.5nM) and HER2 antagonist Lapatnib (Lap, 10nM) additively inhibited proliferation of the HR3+/HER2+ breast cancer cell line BT474 (**Figure 24N**). In control experiments, no inhibition was observed with ER-antagonist ICI 182,780 in ER-negative HR2 MDA-MB-453 cells (**Figure 24O**) or with VDR agonist Calcitrol in VDR-negative (BT549) control cell lines (**Figure 24P**).

Since nearly ninety-five percent of HER2 tumors express at least one hormone receptor, and 29% express all three hormone receptors, these results indicate that hormone treatment might also be possible in a majority of HER2 tumors in combination with anti-HER2 therapy.

Since many modifications, variations and changes in detail can be made to the described preferred embodiment of the invention, it is intended that all matters in the foregoing description and shown in the accompanying drawings be interpreted as illustrative and not in a limiting sense. Thus, the scope of the invention should be determined by the appended claims and their legal equivalents.

### REFERENCES

1. The International Agency for Research on Cancer, S.S., E. Campo, N. Lee Harris, E.S. Jaffe, S.A. Pileri, H. Stein, J. Thiele, J.W. Vardiman. 2008. WHO Classification of Tumours of Haematopoietic and Lymphoid Tissue (IARC WHO Classification of Tumours). World Health Organization.
2. Hamilton, A., Gallipoli, P., Nicholson, E., and Holyoake, T.L. 2010. Targeted therapy in haematological malignancies. J Pathol 220:404-418.
3. Wood, G.S., and Warnke, R.A. 1982. The immunologic phenotyping of bone marrow biopsies and aspirates: frozen section techniques. Blood 59:913-922.
4. Drexler, H.G. 1987. Classification of acute myeloid leukemias--a comparison of FAB and immunophenotyping. Leukemia 1:697-705.
5. Mason, D.Y., and Gatter, K.C. 1987. The role of immunocytochemistry in diagnostic pathology. J Clin Pathol 40:1042-1054.
6. Jones, C., Mackay, A., Grigoriadis, A., Cossu, A., Reis-Filho, J.S., Fulford, L., Dexter, T., Davies, S., Bulmer, K., Ford, E., et al. 2004. Expression profiling of purified normal human luminal and myoepithelial breast cells: identification of novel prognostic markers for breast cancer. Cancer Res 64:3037-3045.
7. Gusterson, B.A., Ross, D.T., Heath, V.J., and Stein, T. 2005. Basal cytokeratins and their relationship to the cellular origin and functional classification of breast cancer. Breast Cancer Res 7:143-148.
8. Reis-Filho, J.S., and Pusztai, L. 2011. Gene expression profiling in breast cancer: classification, prognostication, and prediction. Lancet 378:1812-1823.
9. Nakshatri, H., Srour, E.F., and Badve, S. 2009. Breast cancer stem cells and intrinsic subtypes: controversies rage on. Curr Stem Cell Res Ther 4:50-60.
10. Karantza, V. 2011. Keratins in health and cancer: more than mere epithelial cell markers. Oncogene 30:127-138.
11. Chu, P.G., and Weiss, L.M. 2002. Keratin expression in human tissues and neoplasms. Histopathology 40:403-439.
12. Ince, T.A., Richardson, A.L., Bell, G.W., Saitoh, M., Godar, S., Karnoub, A.E., Iglehart, J.D., and Weinberg, R.A. 2007. Transformation of different human breast epithelial cell types leads to distinct tumor phenotypes. Cancer Cell 12:160-170.
13. Van Keymeulen, A., Rocha, A.S., Ousset, M., Beck, B., Bouvencourt, G., Rock, J., Sharma, N., Dekoninck, S., and Blanpain, C. 2011. Distinct stem cells contribute to mammary gland development and maintenance. Nature 479:189-193.
14. Molyneux, G., Geyer, F.C., Magnay, F.A., McCarthy, A., Kendrick, H., Natrajan, R., Mackay, A., Grigoriadis, A., Tutt, A., Ashworth, A., et al. 2010. BRCA1 basal-like breast cancers originate from luminal epithelial progenitors and not from basal stem cells. Cell Stem Cell 7:403-417.
15. Conzen, S.D. 2008. Minireview: nuclear receptors and breast cancer. Mol Endocrinol 22:2215-2228.
16. Ginty, F., Adak, S., Can, A., Gerdes, M., Larsen, M., Cline, H., Filkins, R., Pang, Z., Li, Q., and Montalto, M.C. 2008. The relative distribution of membranous and cytoplasmic met is a prognostic indicator in stage I and II colon cancer. Clin Cancer Res 14:3814-3822.
17. Maddison, W.P., and Maddison, D.R. 2011. Mesquite: a modular system for evolutionary analysis. Version 2.75 http://mesquiteproject.org.
18. Collins, L.C., Martyniak, A., Kandel, M.J., Stadler, Z.K., Masciari, S., Miron, A., Richardson, A.L., Schnitt, S.J., and Garber, J.E. 2009. Basal cytokeratin and epidermal growth factor receptor expression are not predictive of BRCA1 mutation status in women with triple-negative breast cancers. Am J Surg Pathol 33:1093-1097.
19. Boecker, W., and Buerger, H. 2003. Evidence of progenitor cells of glandular and myoepithelial cell lineages in the human adult female breast epithelium: a new progenitor (adult stem) cell concept. Cell Prolif 36 Suppl 1:73-84.
20. Molyneux, G., and Smalley, M.J. 2011. The cell of origin of BRCA1 mutation-associated breast cancer: a cautionary tale of gene expression profiling. J Mammary Gland Biol Neoplasia 16:51-55.
21. Lavasani, M.A., and Moinfar, F. 2012. Molecular classification of breast carcinomas with particular emphasis on "basal-like" carcinoma: a critical review. J Biophotonics 5:345-366.
22. Livasy, C.A., Karaca, G., Nanda, R., Tretiakova, M.S., Olopade, O.I., Moore, D.T., and Perou, C.M. 2006. Phenotypic evaluation of the basal-like subtype of invasive breast carcinoma. Mod Pathol 19:264-271.
23. Sorlie, T., Perou, C.M., Tibshirani, R., Aas, T., Geisler, S., Johnsen, H., Hastie, T., Eisen, M.B., van de Rijn, M., Jeffrey, S.S., et al. 2001. Gene expression patterns of breast carcinomas distinguish tumor subclasses with clinical implications. Proc Natl Acad Sci U S A 98:10869-10874.
24. Sorlie, T., Tibshirani, R., Parker, J., Hastie, T., Marron, J.S., Nobel, A., Deng, S., Johnsen, H., Pesich, R., Geisler, S., et al. 2003. Repeated observation of breast tumor subtypes in independent gene expression data sets. Proc Natl Acad Sci U S A 100:8418-8423.
25. Grigoriadis, A., Mackay, A., Reis-Filho, J.S., Steele, D., Iseli, C., Stevenson, B.J., Jongeneel, C.V., Valgeirsson, H., Fenwick, K., Iravani, M., et al. 2006. Establishment of the epithelial-specific transcriptome of normal and malignant human breast cells based on MPSS and array expression data. Breast Cancer Res 8:R56.
26. Lakhani, S.R., Reis-Filho, J.S., Fulford, L., Penault-Llorca, F., van der Vijver, M., Parry, S., Bishop, T., Benitez, J., Rivas, C., Bignon, Y.J., et al. 2005. Prediction of BRCA1 status in patients with breast cancer using estrogen receptor and basal phenotype. Clin Cancer Res 11:5175-5180.
27. Raouf, A., Zhao, Y., To, K., Stingl, J., Delaney, A., Barbara, M., Iscove, N., Jones, S., McKinney, S., Emerman, J., et al. 2008. Transcriptome analysis of the normal human mammary cell commitment and differentiation process. Cell Stem Cell 3:109-118.
28. Richardson, A.L., Wang, Z.C., De Nicolo, A., Lu, X., Brown, M., Miron, A., Liao, X., Iglehart, J.D., Livingston, D.M., and Ganesan, S. 2006. X chromosomal abnormalities in basal-like human breast cancer. Cancer Cell 9:121-132.
29. Loi, S., Haibe-Kains, B., Desmedt, C., Wirapati, P., Lallemand, F., Tutt, A.M., Gillet, C., Ellis, P., Ryder, K., Reid, J.F., et al. 2008. Predicting prognosis using molecular profiling in estrogen receptor-positive breast cancer treated with tamoxifen. BMC Genomics 9:239.
30. Ivshina, A.V., George, J., Senko, O., Mow, B., Putti, T.C., Smeds, J., Lindahl, T., Pawitan, Y., Hall, P., Nordgren, H., et al. 2006. Genetic reclassification of histologic grade delineates new clinical subtypes of breast cancer. Cancer Res 66:10292-10301.
31. Desmedt, C., Piette, F., Loi, S., Wang, Y., Lallemand, F., Haibe-Kains, B., Viale, G., Delorenzi, M., Zhang, Y., d'Assignies, M.S., et al. 2007. Strong time dependence of the 76-gene prognostic signature for node-negative breast cancer patients in the TRANSBIG multicenter independent validation series. Clin Cancer Res 13:3207-3214.
32. Lim, E., Vaillant, F., Wu, D., Forrest, N.C., Pal, B., Hart, A.H., Asselin-Labat, M.L., Gyorki, D.E., Ward, T., Partanen, A., et al. 2009. Aberrant luminal progenitors as the candidate target population for basal tumor development in BRCA1 mutation carriers. Nat Med 15:907-913.
33. Gusterson, B. 2009. Do 'basal-like' breast cancers really exist? Nat Rev Cancer 9:128-134.
34. Collins, L.C., Cole, K.S., Marotti, J.D., Hu, R., Schnitt, S.J., and Tamimi, R.M. 2011. Androgen receptor expression in breast cancer in relation to molecular phenotype: results from the Nurses' Health Study. Mod Pathol 24:924-931.
35. Hu, R., Dawood, S., Holmes, M.D., Collins, L.C., Schnitt, S.J., Cole, K., Marotti, J.D., Hankinson, S.E., Colditz, G.A., and Tamimi, R.M. 2011. Androgen receptor expression and breast cancer survival in postmenopausal women. Clin Cancer Res 17:1867-1874.
36. Santagata, S., Hu, R., Lin, N.U., Mendillo, M.L., Collins, L.C., Hankinson, S.E., Schnitt, S.J., Whitesell, L., Tamimi, R.M., Lindquist, S., et al. 2011. High levels of nuclear heat-shock factor 1 (HSF1) are associated with poor prognosis in breast cancer. Proc Natl Acad Sci U S A 108:18378-18383.
37. Harrell, J.C., Prat, A., Parker, J.S., Fan, C., He, X., Carey, L., Anders, C., Ewend, M., and Perou, C.M. 2012. Genomic analysis identifies unique signatures predictive of brain, lung, and liver relapse. Breast Cancer Res Treat 132:523-535.
38. Neve, R.M., Chin, K., Fridlyand, J., Yeh, J., Baehner, F.L., Fevr, T., Clark, L., Bayani, N., Coppe, J.P., Tong, F., et al. 2006. A collection of breast cancer cell lines for the study of functionally distinct cancer subtypes. Cancer Cell 10:515-527.
39. Curtis, C., Shah, S.P., Chin, S.F., Turashvili, G., Rueda, O.M., Dunning, M.J., Speed, D., Lynch, A.G., Samarajiwa, S., Yuan, Y., et al. 2012. The genomic and transcriptomic architecture of 2,000 breast tumours reveals novel subgroups. Nature 486:346-352.
40. Banerji, S., Cibulskis, K., Rangel-Escareno, C., Brown, K.K., Carter, S.L., Frederick, A.M., Lawrence, M.S., Sivachenko, A.Y., Sougnez, C., Zou, L., et al. 2012. Sequence analysis of mutations and translocations across breast cancer subtypes. Nature 486:405-409.
41. Stephens, P.J., McBride, D.J., Lin, M.L., Varela, I., Pleasance, E.D., Simpson, J.T., Stebbings, L.A., Leroy, C., Edkins, S., Mudie, L.J., et al. 2009. Complex landscapes of somatic rearrangement in human breast cancer genomes. Nature 462:1005-1010.
42. TCGA. 2012. Comprehensive molecular portraits of human breast tumours. Nature 490:61-70.
43. Ni, M., Chen, Y., Lim, E., Wimberly, H., Bailey, S.T., Imai, Y., Rimm, D.L., Shirley Liu, X., and Brown, M. 2011. Targeting androgen receptor in estrogen receptor-negative breast cancer. Cancer Cell 20:119-131.
44. MacConaill, L.E., Campbell, C.D., Kehoe, S.M., Bass, A.J., Hatton, C., Niu, L., Davis, M., Yao, K., Hanna, M., Mondal, C., et al. 2009. Profiling critical cancer gene mutations in clinical tumor samples. PLoS One 4:e7887.
45. Yalcin-Ozuysal, O., and Brisken, C. 2009. From normal cell types to malignant phenotypes. Breast Cancer Res 11:306.
46. Godar, S., Ince, T.A., Bell, G.W., Feldser, D., Donaher, J.L., Bergh, J., Liu, A., Miu, K., Watnick, R.S., Reinhardt, F., et al. 2008. Growth-inhibitory and tumor-suppressive functions of p53 depend on its repression of CD44 expression. Cell 134:62-73.
47. McAllister, S.S., Gifford, A.M., Greiner, A.L., Kelleher, S.P., Saelzler, M.P., Ince, T.A., Reinhardt, F., Harris, L.N., Hylander, B.L., Repasky, E.A., et al. 2008. Systemic endocrine instigation of indolent tumor growth requires osteopontin. Cell 133:994-1005.
48. Vogelstein, B., and Kinzler, K.W. 2004. Cancer genes and the pathways they control. Nat Med 10:789-799.
49. Gupta, G.P., and Massague, J. 2006. Cancer metastasis: building a framework. Cell 127:679-695.
50. Tamimi, R.M., Baer, H.J., Marotti, J., Galan, M., Galaburda, L., Fu, Y., Deitz, A.C., Connolly, J.L., Schnitt, S.J., Colditz, G.A., et al. 2008. Comparison of molecular phenotypes of ductal carcinoma in situ and invasive breast cancer. Breast Cancer Res 10:R67.
51. Panchision, D.M., Chen, H.L., Pistollato, F., Papini, D., Ni, H.T., and Hawley, T.S. 2007. Optimized flow cytometric analysis of central nervous system tissue reveals novel functional relationships among cells expressing CD133, CD15, and CD24. Stem Cells 25:1560-1570.
52. Kordek, R., Potemski, P., Kusinska, R., Pluciennik, E., and Bednarek, A. 2010. Basal keratin expression in breast cancer by quantification of mRNA and by immunohistochemistry. J Exp Clin Cancer Res 29:39.
53. Ince, T.A., Ward, J.M., Valli, V.E., Sgroi, D., Nikitin, A.Y., Loda, M., Griffey, S.M., Crum, C.P., Crawford, J.M., Bronson, R.T., et al. 2008. Do-it-yourself (DIY) pathology. Nat Biotechnol 26:978-979; discussion 979.
54. Lu, X., Wang, Z.C., Iglehart, J.D., Zhang, X., and Richardson, A.L. 2008. Predicting features of breast cancer with gene expression patterns. Breast Cancer Res Treat 108:191-201.
55. Birrell, S.N., Bentel, J.M., Hickey, T.E., Ricciardelli, C., Weger, M.A., Horsfall, D.J., and Tilley, W.D. 1995. Androgens induce divergent proliferative responses in human breast cancer cell lines. J Steroid Biochem Mol Biol 52:459-467.
56. Naderi, A., and Hughes-Davies, L. 2008. A functionally significant cross-talk between androgen receptor and ErbB2 pathways in estrogen receptor negative breast cancer. Neoplasia 10:542-548.
57. Emde, A., Mahlknecht, G., Maslak, K., Ribba, B., Sela, M., Possinger, K., and Yarden, Y. 2011. Simultaneous Inhibition of Estrogen Receptor and the HER2 Pathway in Breast Cancer: Effects of HER2 Abundance. Transl Oncol 4:293-300.
58. Dawood, S., Hu, R., Homes, M.D., Collins, L.C., Schnitt, S.J., Connolly, J., Colditz, G.A., and Tamimi, R.M. 2011. Defining breast cancer prognosis based on molecular phenotypes: results from a large cohort study. Breast Cancer Res Treat 126:185-192.
59. Gerner, M.Y., Kastenmuller, W., Ifrim, I., Kabat, J., and Germain, R.N. 2012. Histo-cytometry: a method for highly multiplex quantitative tissue imaging analysis applied to dendritic cell subset microanatomy in lymph nodes. Immunity 37:364-376.
60. Robertson, D., Savage, K., Reis-Filho, J.S., and Isacke, C.M. 2008. Multiple immunofluorescence labelling of formalin-fixed paraffin-embedded (FFPE) tissue. BMC Cell Biol 9:13.
61. Tsurui, H., Nishimura, H., Hattori, S., Hirose, S., Okumura, K., and Shirai, T. 2000. Seven-color fluorescence imaging of tissue samples based on Fourier spectroscopy and singular value decomposition. J Histochem Cytochem 48:653-662.
62. Shi, S.R., Shi, Y., and Taylor, C.R. 2011. Antigen retrieval immunohistochemistry: review and future prospects in research and diagnosis over two decades. J Histochem Cytochem 59:13-32.
63. Glass, G., Papin, J.A., and Mandell, J.W. 2009. SIMPLE: a sequential immunoperoxidase labeling and erasing method. J Histochem Cytochem 57:899-905.

## Claims

1. A method of classifying breast cancer, comprising:
measuring the level of estrogen receptor, ER, expressed in cancerous breast tissue obtained from a patient,
measuring the level of androgen receptor, AR, expressed in the cancerous breast tissue obtained from the patient,
measuring the level of vitamin D receptor, VDR, expressed in the cancerous breast tissue obtained from the patient,
classifying, in a first category HR0, a breast cancer tumor defined by a substantial lack of ER , AR, and VDR expression in the breast cancer tumor;
classifying, in a second category HR1, a breast cancer tumor defined by expression of only one of ER, AR, and VDR in the breast cancer tumor;
classifying, in a third category HR2, a breast cancer tumor defined by expression of any combination of two of ER, AR, and VDR in a breast cancer tumor; and
classifying, in a fourth category HR3, a breast cancer tumor defined by expression of each of ER, AR, and VDR in the breast cancer tumor, and
classifying the breast cancer tumor of the patient as HR0, HR1, HR2, or HR3 based on the expression levels of ER, AR, and VDR measured in the cancerous breast tissue,
wherein tumors of said first category HR0, said second category HR1, said third category HR2, and said fourth category HR3 correspond to significant survival differences in patients.

2. The method as recited in claim 1, wherein said first category HR0 is defined as having less than about 1% of tumor cells, or less than about 5% of tumor cells, or less than about 10% of tumor cells, or less than about 30% of tumor cells, or less than about 50% of tumor cells, with expression of ER, AR, and VDR in the tumor.

3. The method as recited in claim 1, wherein expression levels of ER, AR, and VDR in tumors of said first category HR0, said second category HR1, said third category HR2, and said fourth category HR3 correspond to levels of expression of ER, AR, and VDR in normal breast tissue.

4. The method as recited in claim 1 further comprising measuring the level of human epidermal growth factor receptor, HER2, expressed in the cancerous breast tissue of the patient.

5. The method as recited in claim 1, further comprising predicting a prognosis of breast cancer by:
determining a higher survival rate when the tumor expresses all of ER, AR, and VDR;
determining an intermediate survival rate if the tumor expresses only two of ER, AR, and VDR; or
determining a lower survival rate if the tumor expresses only one of ER, AR, and VDR.

6. The method as recited in claim 1, further comprising determining a treatment regimen for a breast cancer patient by:
including in the treatment regimen at least one ER antagonist ligand when the cancerous breast tissue expresses ER;
including in the treatment regimen at least one AR ligand when the cancerous breast tissue expresses AR; and
including in the treatment regimen at least one VDR agonist ligand when the cancerous breast tissue expresses VDR.

7. The method as recited in claim 4, further comprising determining a treatment regimen for a breast cancer patient by:
including in the treatment regimen at least one ER antagonist ligand when the cancerous breast tissue expresses ER;
including in the treatment regimen at least one AR ligand when the cancerous breast tissue expresses AR;
including in the treatment regimen at least one VDR agonist ligand when the cancerous breast tissue expresses VDR; and
including in the treatment regimen at least one HER2 inhibitor when the cancerous breast tissue expresses HER2.

8. Use of a kit in a method as set forth in any one of claims 1-7, the kit comprising:
a predetermined amount of a first probe capable of binding to estrogen receptor, ER, protein or messenger ribonucleic acid, mRNA, or detecting ER activity;
a predetermined amount of a second probe capable of binding to androgen receptor, AR, protein or mRNA, or detecting AR activity, and
a predetermined amount of a third probe capable of binding to vitamin D receptor, VDR, protein or mRNA, or detecting VDR activity,
wherein each of the probes are for detecting protein, or each of the probes are for detecting mRNA, or each of the probes are for detecting activity.

9. Use of the kit according to claim 8, the kit further comprising a predetermined amount of a fourth probe capable of binding to human epidermal growth factor receptor 2, HER2, protein or mRNA, or detecting HER2 activity.

10. Use of the kit according to claim 8 or 9, the kit further comprising a predetermined amount of at least one detection probe to permit the detection of any of said first probe, said second probe, said third probe, and said optional fourth probe.

11. Use of the kit according to any one of claims 8 to 10, the kit being comprised of:
a predetermined amount of primary antibody capable of binding at least one binding site of ER, a predetermined amount of primary antibody capable of binding at least one binding site of AR, and a predetermined amount of primary antibody capable of binding at least one binding site of VDR; optionally a predetermined amount of primary antibody capable of binding at least one binding site of HER2; and optionally a predetermined amount of secondary antibody capable of binding at least one of said primary antibody for ER, AR, and VDR, wherein the probes comprise antibodies for detecting said proteins.

## Patentansprüche

1. Verfahren zum Klassifizieren von Brustkrebs, welches Folgendes aufweist:
Messen des Niveaus eines Estrogenrezeptors ER, der in krebsartigem Brustgewebe exprimiert bzw. ausgeprägt ist, welches von einem Patienten erhalten wurde,
Messen des Niveaus eines Androgenrezeptors AR, der in dem krebsartigen Brustgewebe exprimiert ist, welches von dem Patienten erhalten wurde,
Messen des Niveaus eines Vitamin-D-Rezeptors VDR, der in dem krebsartigen Brustgewebe exprimiert ist, das von dem Patienten erhalten wurde,
Klassifizieren eines Brustkrebstumors, der durch einen wesentlichen Mangel an ER-, AR- und VDR-Expression in dem Brustkrebstumor definiert ist, in einer ersten Kategorie HR0;
Klassifizieren eines Brustkrebstumors, der durch eine Expression von nur einem von ER, AR und VDR in dem Brustkrebstumor definiert ist, in einer zweiten Kategorie HR1;
Klassifizieren eines Brustkrebstumors, der durch eine Expression von irgendeiner Kombination von zwei von ER, AR und VDR in einem Brustkrebstumor definiert ist, in einer dritten Kategorie HR2; und
Klassifizieren eines Brustkrebstumors, der durch eine Expression von jedem von ER, AR und VDR in dem Brustkrebstumor definiert ist, in einer vierten Kategorie HR3; und
Klassifizieren des Brustkrebstumors des Patienten als HR0, HR1, HR2 oder HR3 basierend auf den Expressionsniveaus von ER, AR und VDR, die in dem krebsartigen Brustgewebe gemessen wurden, wobei Tumore der ersten Kategorie HR0, der zweiten Kategorie HR1, der dritten Kategorie HR2 und der vierten Kategorie HR3 signifikanten Überlebensunterschieden bei den Patienten entsprechen.

2. Verfahren nach Anspruch 1, wobei die erste Kategorie HR0 derart definiert ist, dass sie weniger als ungefähr 1% Tumorzellen oder weniger als ungefähr 5% Tumorzellen oder weniger als ungefähr 10% Tumorzellen oder weniger als ungefähr 30% Tumorzellen oder weniger als ungefähr 50% Tumorzellen mit Expression von ER, AR und VDR in dem Tumor hat.

3. Verfahren nach Anspruch 1, wobei die Expressionsniveaus von ER, AR und VDR in Tumoren der ersten Kategorie HR0, der zweiten Kategorie HR1, der dritten Kategorie HR2 und der vierten Kategorie HR3 Expressionsniveaus von ER, AR und VDR in normalem Brustgewebe entsprechen.

4. Verfahren nach Anspruch 1, welches weiter aufweist, das Niveau des menschlichen Epidermalen Wachstumsfaktorrezeptors HER2 zu messen, welches in dem krebsartigen Brustgewebe des Patienten exprimiert, bzw. ausgeprägt ist.

5. Verfahren nach Anspruch 1, welches weiter aufweist, eine Prognose für Brustkrebs vorherzusagen durch:
Bestimmen einer höheren Überlebensrate, wenn der Tumor alle von ER, AR und VDR exprimiert;
Bestimmen einer mittleren Überlebensrate bzw.
Zwischenüberlebensrate, wenn der Tumor nur zwei von ER, AR und VDR exprimiert; oder
Bestimmen einer geringeren Überlebensrate, wenn der Tumor nur eines von ER, AR und VDR exprimiert.

6. Verfahren nach Anspruch 1, welches weiter aufweist, einen Behandlungsplan für einen Brustkrebspatienten zu bestimmen durch:
Einschließen mindestens eines ER-Antagonist-Liganden in den Behandlungsplan, wenn das krebsartige Brustgewebe ER exprimiert;
Einschließen mindestens eines AR-Liganden in den Behandlungsplan, wenn das krebsartiges Brustgewebe AR exprimiert; und
Einschließen mindestens eines VDR-Antagonist-Liganden in den Behandlungsplan, wenn das krebsartige Brustgewebe VDR exprimiert.

7. Verfahren nach Anspruch 4, welches weiter aufweist, einen Behandlungsplan für einen Brustkrebspatienten zu bestimmen durch:
Einschließen von mindestens einem ER-Antagonist-Liganden in den Behandlungsplan, wenn das krebsartige Brustgewebe ER exprimiert;
Einschließen von mindestens einem AR-Liganden in den Behandlungsplan, wenn das krebsartige Brustgewebe AR exprimiert;
Einschließen von mindestens einem VDR-Antagonist-Liganden in den Behandlungsplan, wenn das krebsartige Brustgewebe VDR exprimiert; und
Einschließen mindestens eines HER2-Inhibitors in den Behandlungsplan, wenn das krebsartige Brustgewebe HER2 exprimiert.

8. Verwendung einer Ausrüstung bei einem Verfahren nach einem der Ansprüche 1-7, wobei die Ausrüstung Folgendes aufweist:
eine vorbestimmte Menge eines ersten Testers, der fähig ist, an ein Estrogenrezeptor- bzw. ER-Protein oder eine ER-Messenger-Ribonukleinsäure bzw. ER-mRNA zu binden, oder ER-Aktivität zu detektieren;
eine vorbestimmte Menge eines zweiten Testers, der fähig ist, an ein Androgenrezeptor- bzw. AR-Protein oder an AR-mRNA zu binden oder AR-Aktivität zu detektieren und
eine vorbestimmte Menge eines dritten Testers, der fähig ist, an ein Vitamin-D-Rezeptor- bzw. VDR-Protein oder VDR-mRNA zu binden oder VDR-Aktivität zu detektieren,
wobei jeder der Tester zum Detektieren von Protein ist, oder wobei jeder der Tester zum Detektieren von mRNA ist, oder wobei jeder der Tester zum Detektieren einer Aktivität ist.

9. Verwendung der Ausrüstung nach Anspruch 8, wobei die Ausrüstung weiter eine vorbestimmte Menge eines vierten Testers aufweist, der fähig ist, an ein menschliches Epidermales Wachstumsfaktorrezeptor-2- bzw. HER2-Protein oder HER2-mRNA zu binden, oder eine HER2-Aktivität zu detektieren.

10. Verwendung der Ausrüstung nach Anspruch 8 oder 9, wobei die Ausrüstung weiter eine vorbestimmte Menge von mindestens einem Detektionstester aufweist, um die Detektion von irgendeinem des ersten Testers, des zweiten Testers, des dritten Testers und des optionalen vierten Testers zu gestatten.

11. Verwendung der Ausrüstung nach einem der Ansprüche 8-10, wobei die Ausrüstung aus Folgendem besteht:
eine vorbestimmte Menge eines primären Antikörpers, der fähig ist, zumindest an eine Bindungsstelle von ER zu binden, eine vorbestimmte Menge eines primären Antikörpers, der fähig ist, an mindestens eine Bindungsstelle von AR zu binden, und eine vorbestimmte Menge eines primären Antikörpers, der fähig ist, an mindestens eine Bindungsstelle von VDR zu binden; optional eine vorbestimmte Menge eines primären Antikörpers, der fähig ist, an zumindest eine Bindungsstelle von HER2 zu binden; und optional eine vorbestimmte Menge eines sekundären Antikörpers, der fähig ist, an zumindest einen der primären Antikörper für ER, AR und VDR zu binden, wobei die Tester Antikörper zum Detektieren der Proteine aufweisen.

## Revendications

1. Procédé de classification d'un cancer du sein, comprenant les étapes suivantes :
la mesure du niveau de récepteur des oestrogènes, ER, exprimé dans des tissus mammaires cancéreux obtenus sur un patient,
la mesure du niveau de récepteur des androgènes, AR, exprimé dans les tissus mammaires cancéreux obtenus sur le patient,
la mesure du niveau de récepteur de vitamine D, VDR, exprimé dans les tissus mammaires cancéreux obtenus sur le patient,
la classification, dans une première catégorie HR0, d'une tumeur d'un cancer du sein définie par un déficit substantiel d'expression d'ER, d'AR, et de VDR dans la tumeur d'un cancer du sein ;
la classification, dans une deuxième catégorie HR1, d'une tumeur d'un cancer du sein définie par l'expression d'un seul parmi ER, AR, et VDR dans la tumeur d'un cancer du sein ;
la classification, dans une troisième catégorie HR2, d'une tumeur d'un cancer du sein définie par l'expression d'une quelconque combinaison de deux parmi ER, AR, et VDR dans une tumeur d'un cancer du sein ; et
la classification, dans une quatrième catégorie HR3, d'une tumeur d'un cancer du sein définie par l'expression de chacun de ER, de AR, et de VDR dans la tumeur d'un cancer du sein, et
la classification de la tumeur d'un cancer du sein du patient en tant que HR0, HR1, HR2, ou HR3 sur la base des niveaux d'expression de ER, de AR, et de VDR mesurés dans les tissus mammaires cancéreux,
dans lequel des tumeurs de ladite première catégorie HR0, de ladite deuxième catégorie HR1, de ladite troisième catégorie HR2, et de ladite quatrième catégorie HR3 correspondent à des différences de survie significatives chez les patients.

2. Procédé selon la revendication 1, dans lequel ladite première catégorie HR0 est définie comme ayant moins d'environ 1 % de cellules tumorales, ou moins d'environ 5 % de cellules tumorales, ou moins d'environ 10 % de cellules tumorales, ou moins d'environ 30 % de cellules tumorales, ou moins d'environ 50 % de cellules tumorales, avec une expression de ER, de AR, et de VDR dans la tumeur.

3. Procédé selon la revendication 1, dans lequel des niveaux d'expression de ER, de AR, et de VDR dans des tumeurs de ladite première catégorie HR0, de ladite deuxième catégorie HR1, de ladite troisième catégorie HR2, et de ladite quatrième catégorie HR3 correspondent à des niveaux d'expression de ER, de AR, et de VDR dans des tissus mammaires normaux.

4. Procédé selon la revendication 1, comprenant en outre la mesure du niveau de récepteur du facteur de croissance de l'épiderme humain, HER2, exprimé dans les tissus mammaires cancéreux du patient.

5. Procédé selon la revendication 1, comprenant en outre la prédiction d'un pronostic de cancer du sein en :
déterminant un taux de survie plus élevé quand la tumeur exprime tous les ER, AR, et VDR ;
déterminant un taux de survie intermédiaire si la tumeur exprime seulement deux parmi ER, AR, et VDR ; ou en
déterminant un taux de survie plus faible si la tumeur exprime un seul parmi ER, AR, et VDR.

6. Procédé selon la revendication 1, comprenant en outre la détermination d'un schéma de traitement pour un patient atteint d'un cancer du sein, en :
incorporant dans le schéma de traitement au moins un antagoniste de l'ER quand les tissus mammaires cancéreux expriment l'ER ;
incorporant dans le schéma de traitement au moins un ligand d'AR quand les tissus mammaires cancéreux expriment l'AR ; et
incorporant dans le schéma de traitement au moins un ligand agoniste de VDR quand les tissus mammaires cancéreux expriment le VDR.

7. Procédé selon la revendication 4, comprenant en outre la détermination d'un schéma de traitement pour un patient atteint d'un cancer du sein, en :
incorporant dans le schéma de traitement au moins un ligand antagoniste de l'ER quand les tissus mammaires cancéreux expriment l'ER ;
incorporant dans le schéma de traitement au moins un ligand de l'AR quand les tissus mammaires cancéreux expriment l'AR ;
incorporant dans le schéma de traitement au moins un ligand agoniste du VDR quand les tissus mammaires cancéreux expriment le VDR ; et en
incorporant dans le schéma de traitement au moins un inhibiteur de HER2 quand les tissus mammaires cancéreux expriment HER2.

8. Utilisation d'un kit dans un procédé selon l'une quelconque des revendications 1 à 7, le kit comprenant :
une quantité prédéterminée d'une première sonde capable de se lier à la protéine du récepteur d'oestrogène, ER, ou à un acide ribonucléique messager, ARNm, ou de détecter une activité ER ;
une quantité prédéterminée d'une deuxième sonde capable de se lier à la protéine du récepteur d'androgène, AR, ou à ARNm, ou de détecter une activité AR, et
une quantité prédéterminée d'une troisième sonde capable de se lier à la protéine du récepteur de la vitamine D, VDR, ou à ARNm, ou de détecter une activité VDR,
dans lequel chacune des sondes permet de détecter une protéine, ou chacune des sondes permet de détecter un ARNm, ou chacune des sondes permet de détecter une activité.

9. Utilisation du kit selon la revendication 8, le kit comprenant en outre une quantité prédéterminée d'une quatrième sonde capable de se lier à la protéine du récepteur 2 de facteur de croissance de l'épiderme humain, HER2, ou un ARNm, ou de détecter une activité HER2.

10. Utilisation du kit selon la revendication 8 ou 9, le kit comprenant en outre une quantité prédéterminée d'au moins une sonde de détection pour permettre la détection d'une quelconque parmi ladite première sonde, ladite deuxième sonde, ladite troisième sonde et ladite quatrième sonde optionnelle.

11. Utilisation du kit selon l'une quelconque des revendications 8 à 10, le kit étant constitué de :
une quantité prédéterminée d'anticorps primaire capable de se lier à au moins un site de liaison de l'ER, une quantité prédéterminée d'anticorps primaire capable de se lier à au moins un site de liaison de l'AR, et une quantité prédéterminée d'anticorps primaire capable de se lier à au moins un site de liaison du VDR ; optionnellement une quantité prédéterminée d'anticorps primaire capable de se lier à au moins un site de liaison de HER2 ; et optionnellement une quantité prédéterminée d'anticorps secondaire capable de se lier à au moins un parmi lesdits anticorps primaires pour ER, AR, et VDR, dans lequel les sondes comprennent des anticorps pour détecter lesdites protéines.
